# EUROPEAN PATENT APPLICATION

(11) **EP 4 282 437 A1**
(43) Date of publication of application: **29.11.2023**
(21) Application number: 22382507.6
(22) Date of filing: 26.05.2022
(51) Int. Cl.: A61K 49/18, B82Y 5/00

(54) **DYSPROSIUM NANOPARTICLES, PROCESS FOR OBTAINING SAID NANOPARTICLES AND USE OF SAID NANOPARTICLES AS A CONTRAST AGENT**

(71) Applicant: Consejo Superior De Investigaciones Científicas - CSIC, 41013 Sevilla (ES); Servicio Andaluz de Salud, 41071 Sevilla (ES)
(72) Inventor: Ocaña Jurado,, Manuel, 41013 SEVILLA (ES); Becerro Nieto, Ana Isabel, 41013 SEVILLA (ES); Núñez Álvarez, Nuria Ofelia, 41013 SEVILLA (ES); Gómez González, Elisabet, 41013 SEVILLA (ES); García Martín, Marisa, 41071 SEVILLA (ES); Caro Salazar, Carlos, 41071 SEVILLA (ES)
(74) Representative: Pons

(57) **Abstract**

The invention relates to nanoparticles comprising a core consisting of a dysprosium compound, said dysprosium compound being selected from DyPO₄ or NaDy(MoO₄)₂, and a coating of polyacrylic acid. Furthermore, the present invention refers to processes for obtaining said nanoparticles and to the uses of the nanoparticles as contrast agents for ultra-high-field magnetic resonance imaging.

## Description

The invention relates to a nanoparticles comprising a core consisting of a dysprosium compound, said dysprosium compound is selected from DyPO₄ or NaDy(MoO₄)₂ and a coating of polyacrylic acid. Furthermore, the present invention refers to the process for obtaining said nanoparticles and to the use of the nanoparticles as a contrast agent particularly for ultra-high-field magnetic resonance imaging.

Therefore, the present invention refers to the medical field and is of interest for the industry and users of magnetic resonance imaging techniques.

### BACKGROUND ART

Magnetic resonance imaging (MRI) is a well-established non-invasive technique commonly used for medical diagnosis. To record MRI images, a powerful magnet is used to create a net magnetization of the endogenous water proton spins in the direction longitudinal to the magnetic field (z), and a radiofrequency current is applied to alter the population balance of this magnetization, shifting it towards the plane transverse to the external magnetic field (xy). Once the current ceases, the net magnetization (Mz) is recovered by a double relaxation process (longitudinal and transverse). The times associated with this relaxation process are called T₁ (for longitudinal relaxation) and T₂ (for transverse relaxation) [Cao, Y.; Xu, L.; Kuang, Y.; Xiong, D.; Pei, R. Gadolinium-based nanoscale MRI contrast agents for tumor imaging. Journal of Materials Chemistry B 2017, 5 (19), 3431-3461]*.* In an MRI scan, a contrast between the different analyzed zones is based on the differences in the density of protons between different tissues and the differences in their longitudinal (T₁) and transverse (T₂) relaxation times [Peng, E.; Wang, F.; Xue, J. M. Nanostructured magnetic nanocomposites as MRI contrast agents. Journal of Materials Chemistry B 2015, 3 (11), 2241-2276]*.* T₁-weighted or T₂-weighted images are obtained by using acquisition schemes that potentiate the effect of T₁ or T₂, respectively.

To increase the contrast between the regions of interest and the surrounding tissue, contrast agents (CAs) consisting of magnetically active substances that shorten the T₁ and T₂ relaxation times of protons are often used. The ability of a CA to shorten T₁ and T₂ is expressed through the longitudinal (*r₁*) and transverse (*r₂*) relaxivity, respectively. In current clinical trials, superparamagnetic iron oxide (SPIO) nanoparticles (NPs) are used as T₂ Cas [Wang, Y.-X. J. Superparamagnetic iron oxide based MRI contrast agents: Current status of clinical application. Quantitative imaging in medicine and surgery 2011, 1 (1), 35-40*]* and Gd³⁺ chelates as T₁ Cas [Pan, D.; Schmieder, A. H.; Wickline, S. A.; Lanza, G. M. Manganese-based MRI contrast agents: past, present, and future. Tetrahedron 2011, 67 (44), 8431-8444*].* Different Gd-based inorganic NPs have also been proposed as MRI CAsc because of their easy functionalization and bioconjugation, which allows targeting, thus reducing the needed doses [Na, H. B.; Song, I. C.; Hyeon, T. Inorganic Nanoparticles for MRI Contrast Agents. Advanced Materials 2009, 21 (21), 2133-2148] and their controlled release through particle size selection [Barreto, J. A.; O'Malley, W.; Kubeil, M.; Graham, B.; Stephan, H.; Spiccia, L. Nanomaterials: Applications in Cancer Imaging and Therapy. Advanced Materials 2011, 23 (12), H18-H40]. For the use of these nanoparticulate contrast agents, they must fulfill certain requirements such as uniform particle shape and size (always below 100 nm to avoid embolism and facilitate the renal release) [Duan, X.; Li, Y. Physicochemical Characteristics of Nanoparticles Affect Circulation, Biodistribution, Cellular Internalization, and Trafficking. Small 2013, 9 (9-10), 1521-1532], high chemical and colloidal stability in physiological media, high biocompatibility (absence of cytotoxicity) and in the case of MRI, high relaxivity.

Another recently proposed strategy to improve magnetic resonance images is to increase the magnetic field strength to values higher than those used today in clinical trials (usually ≤ 3 T), which results in a better signal-to-noise ratio and greater spatial/temporal resolution, thus enhancing the sensitivity of the MRI technique *[*Norek, M.; Kampert, E.; Zeitler, U.; Peters, J. A. Tuning of the size of DY2O3 nanoparticles for optimal performance as an MRI contrast agent. Journal of the American Chemical Society 2008, 130 (15), 5335-5340*].* The MRI CAs mentioned above are not effective at high magnetic fields [Lisjak, D.; Plohl, O.; Vidmar, J.; Majaron, B.; Ponikvar-Svet, M. Dissolution Mechanism of Upconverting AYF(4):Yb,Tm (A = Na or K) Nanoparticles in Aqueous Media. Langmuir 2016, 32 (32), 8222-8229]*.* The most promising candidates are Dy³⁺ containing compounds due to the very high magnetic moment of this cation (10.6 *µB)* and short electronic relaxation times, which favors their *r₂* relaxivity at high field. In response to the high field (≥ 7 T) MRI CAs demand, several Dy³⁺ based systems have been reported, consisting of NaDyF₄ [Zhang, Y.; Vijayaragavan, V.; Das, G. K.; Bhakoo, K. K.; Tan, T. T. Y. Single-Phase NaDyF4:Tb3+ Nanocrystals as Multifunctional Contrast Agents in High-Field Magnetic Resonance and Optical Imaging. European Journal of Inorganic Chemistry 2012, (12), 2044-2048]*,* DyF₃ *[*Gonzalez-Mancebo, D.; Becerro, A. I.; Rojas, T. C.; Garcia-Martin, M. L.; de la Fuente, J. M.; Ocana, M. HoF3 and DyF3 Nanoparticles as Contrast Agents for High-Field Magnetic Resonance Imaging. Particle & Particle Systems Characterization 2017, 34 (10*)*], Dy₂O₃ and DyVO₄ *[*Gomez-Gonzalez, E.; Nunez, N. O.; Caro, C.; Garcia-Martin, M. L.; Fernandez-Afonso, Y.; de la Fuente, J. M.; Balcerzyk, M.; Ocana, M. Dysprosium and Holmium Vanadate Nanoprobes as High- Performance Contrast Agents for High-Field Magnetic Resonance and Computed Tomography Imaging. Inorganic Chemistry 2021, 60 (1), 152-160]. However, flanthanide (Ln) fluorides have been shown to be chemically unstable in aqueous solution, since they slowly dissolve and release potentially toxic ions [Lisjak, D.; Plohl, O.; Vidmar, J.; Majaron, B.; Ponikvar-Svet, M. Dissolution Mechanism of Upconverting AYF(4):Yb,Tm (A = Na or K) Nanoparticles in Aqueous Media. Langmuir 2016, 32 (32), 8222-8229]*.*

Therefore, developing MRI contrast agents that show optimum contrast at high magnetic fields is an urgent task.

### SUMMARY OF THE INVENTION

The present invention discloses novel nanoparticles comprising a core consisting of a dysprosium compound, said dysprosium compound is selected from DyPO₄, or NaDy(MoO₄)₂ and a coating of polyacrylic acid. Said nanoparticles are suitable for use as a contrast agent for ultra-high-field (UHF) magnetic resonance imaging (MRI) working at fields ≥ 7 T.

Therefore, a first aspect of the invention refers to nanoparticles (herein the nanoparticles of the present invention) comprising
- a core consisting of a dysprosium compound, said dysprosium compound is selected from DyPO₄ or NaDy(MoO₄)₂,
- and a coating of polyacrylic acid.

wherein the DyPO₄ nanoparticles are cubic-like and have an average value of the face diagonal ranging from 23 nm to 57 nm, and
wherein the NaDy(MoO₄)₂ nanoparticles
   - are spherical and have a size ranging from 10 nm and 30 nm,
   - or are ellipsoidal and have a first axis ranging from 40 nm to 95 nm and a second axis ranging from 10 nm to 30 nm.

Said nanoparticles exhibit biocompatibility and notably improved colloidal stability in water and in phosphate-buffered saline PBS (a buffer solution commonly used in biological research to simulate physiological fluid). In addition to their colloidal stability in physiological fluid, compounds, materials or particles intended to be applied in nanomedicine should be chemically stable, i.e., nanoparticles dissolution should not take place in the physiological fluid during the period between the injection and the excretion of the nanoparticles to hinder potential toxic ions to be released to the blood stream. The nanoparticles of the present invention show an excellent degree of chemical stability in PBS. Furthermore, the nanoparticles of the present invention exhibit negligible toxicity effects under the assayed conditions, and, therefore, are suitable as bioimaging probes.

A preferred embodiment of the nanoparticles of the present invention refers to nanoparticles having an average value of the face diagonal ranging from 23 nm to 57 and comprising
- a core of cubic-like DyPO₄,
- a first coating of polyacrylic acid on the surface of the core of cubic-like DyPO₄,
- a second coating of polyethylene glycol on the surface of the first coating.

Another preferred embodiment of the nanoparticles of the present invention refers to nanoparticles having a size ranging from 10 nm and 30 nm and comprising
- a core of spherical NaDy(MoO₄)₂
- a first coating of poly(allylamine hydrochloride) positively charged polyelectrolyte on the surface of the core of spherical NaDy(MoO₄)₂
- and a second coating of polyacrylic acid negatively charged polyelectrolyte on the surface of the first coating.

More preferably the spherical NaDy(MoO₄)₂ nanoparticles have a size ranging from 16 nm and 23 nm.

Another preferred embodiment of the nanoparticles of the present invention refers to nanoparticles having a first axis ranging from 40 nm to 95 nm and a second axis ranging from 10 nm to 30 nm, and comprising
- a core of ellipsoidal NaDy(MoO₄)₂,
- tartrate anions adsorbed at the surface of the core of NaDy(MoO₄)₂,
- a first coating of poly(allylamine hydrochloride) positively charged polyelectrolyte on the surface of the tartaric anions,
- and a second coating of polyacrylic acid negatively charged polyelectrolyte on the surface of the first coating,

Preferably the ellipsoidal NaDy(MoO₄)₂ have a first axis ranging from 50 nm to 87 nm and a second axis ranging from 16 nm to 22 nm.

Another aspect of the invention refers to process for obtaining the nanoparticles having an average value of the face diagonal ranging from 23 nm to 57 nm and comprising
- a core of cubic-like DyPO₄ and
- and a coating of polyacrylic acid,
characterized in that it comprises the following steps:
a) aging, in a microwave oven, a mixture of a butanol solution of dysprosium nitrate and phosphoric acid at a temperature ranging between 120 ºC and 180 ºC for a period of time ranging between 45 min and 90 min, wherein the concentration of phosphoric acid is ranging between 0.075 M and 0.30 M in the mixture, and
b) preparing an aqueous suspension of the product obtained in step (a) and adding poly(acrylic acid) in a concentration between 2 mg/ml and 10 mg/ml adjusting the pH to a value ranging between 9 and 11.

Step (a) refers to the aging, in a microwave oven, a mixture of a butanol solution of dysprosium nitrate and phosphoric acid at a temperature ranging from 120 ºC and 180 ºC, preferably between 140 ºC and 160 ºC, for a period of time ranging between 45 min and 90 min, wherein the concentration of phosphoric acid is ranging between 0.075 M and 0.30 M in the mixture. It is observed that at a temperature higher than 180 ºC the size of the nanoparticles increases and at a temperature lower than 120 ºC a precipitation of heterogeneous nanoparticles.

Another aspect of the invention refers to the process for obtaining the nanoparticles having an average value of the face diagonal ranging from 23 nm to 57 and comprising
- a core of cubic-like DyPO₄,
- a first coating of polyacrylic acid on the surface of the core of cubic-like DyPO₄,
- and a second coating of polyethylene glycol on the surface of the first coating, characterized in that it comprises the following steps:
   a) aging, in a microwave oven, a mixture of a butanol solution of dysprosium nitrate and phosphoric acid at a temperature ranging between 120 ºC and 180 ºC, preferably between 140 ºC and 160 ºC, for a period of time ranging between 45 min and 90 min, wherein the concentration of phosphoric acid is ranging between 0.075 M and 0.30 M in the mixture, and
   b) preparing an aqueous suspension of the product obtained in step (a) and adding poly(acrylic acid) in a concentration between 1 mg/ml and 20 mg/ml, preferably in a concentration between 2 mg/ml and 10 mg/ml, adjusting the pH to a value ranging between 9 and 11, and
   c) preparing a suspension of the nanoparticles obtained in step (b) in a borate buffer, wherein said borate buffer comprises an *N*-ethyl-*N'*-(3-(dimethylamino)propyl)carbodiimide (EDC) solution and an *N*-hydroxysuccinimide (s-NHS) solution, and adding a borate buffer PEG solution in a concentration ranging between 5 mg/ml and 20 mg/ml, preferably in a concentration of 10 mg/ml.

In step (b) the concentration of poly(acrylic acid) ranges preferably between 1 mg/ml and 20 mg/ml, more preferably in a concentration between 2 mg/ml and 10 mg/ml, to ensure the formation of an increased number of carboxylate groups on the nanoparticles surface for PEG grafting in step (c). This step (b) is indispensable to create an amide link between carboxylate groups of PAA and the amine group of PEG. The purpose of the PEG functionalization is improving the NPs' systemic circulation time and avoiding their clearance by the immune system.

Another aspect of the invention refers to the process for obtaining the nanoparticles having a size ranging from 10 nm and 30 nm, preferably having a size ranging from 16 nm and 23 nm, and comprising
- a core of spherical NaDy(MoO₄)₂,
- a first coating of poly(allylamine hydrochloride) positive charged polyelectrolyte on the surface of the core of spherical NaDy(MoO₄)₂,
- and a second coating of polyacrylic acid negative charged polyelectrolyte on the surface of the first coating,
characterized in that it comprises the following steps:
a) aging, in a conventional oven, a mixture of an aqueous and ethylene glycol solution of sodium molybdate and an ethylene glycol solution of dysprosium chloride at a temperature ranging between 110 ºC and 130 ºC for a period of time ranging between 12 h and 25 h, wherein the concentration of Dy⁺³ is ranging between 0.01 M and 0.04 M in the mixture, preferably at a concentration of Dy⁺³ of 0.02 M in the mixture, and wherein the concentration of MoO₄²⁻ is ranging between 0.01 M and 0.04 M in the mixture, preferably at a concentration of MoO₄²⁻ of 0.02 M in the mixture.
b) mixing an aqueous solution comprising NaCl aqueous solution and poly(allylamine hydrochloride) with an aqueous dispersion of the nanoparticles obtained in step (a) and sonicating in ice water, and
c) mixing an aqueous solution comprising NaCl aqueous solution and polyacrylic acid with the nanoparticles obtained in step (b) and sonicating in ice water.

Another aspect of the invention refers to the process for obtaining nanoparticles having a first axis ranging from 40 nm to 95 nm and a second axis ranging from 10 nm to 30 nm, preferably having a first axis ranging from 50 nm to 87 nm and a second axis ranging from 16 nm to 22 nm, and comprising
- a core of ellipsoidal NaDy (MoO₄)₂,
- and tartrate anions adsorbed at the surface of the core of ellipsoidal NaDy (MoO₄)₂,
- a first coating of poly(allylamine hydrochloride) positive charged polyelectrolyte on the surface of the tartaric anions,
- and a second coating of polyacrylic acid negative charged polyelectrolyte on the surface of the first coating,
characterized in that it comprises the following steps:
a) aging, in a microwave oven, a mixture of an aqueous and ethylene glycol solution of sodium molybdate, an ethylene glycol solution of dysprosium chloride and tartaric acid at a temperature ranging between 210 ºC and 230 ºC for a period of time ranging between 20 min and 60 min, wherein the concentration of Dy⁺³ is ranging between 0.01 M and 0.04 M in the mixture, preferably at a concentration of Dy⁺³ of 0.02 M in the mixture, wherein the concentration of MoO₄²⁻ is ranging between 0.1 M and 0.4 M in the mixture, preferably at a concentration of M0O₄²⁻ of 0.2 M in the mixture and wherein the concentration of tartaric acid is ranging between 0.01 M and 0.1 M in the mixture, preferably the concentration of tartaric acid is ranging between 0.02 M and 0.06 M in the mixture,
b) mixing an aqueous solution comprising NaCl aqueous solution and poly(allylamine hydrochloride) with an aqueous dispersion of the nanoparticles obtained in step (a) and sonicating in ice water, and
c) mixing an aqueous solution comprising NaCl aqueous solution and polyacrylic acid with the nanoparticles obtained in step (b) and sonicating in ice water.

Another aspect of the present invention relates to the nanoparticles of the present invention for use as a contrast agent for imaging blood flow in magnetic resonance imaging, preferably for use as a contrast agent for imaging blood flow in ultra-high-field magnetic resonance imaging operating at static magnetic fields of 7 T or higher.

Another aspect of the invention refers to the use of the nanoparticles of the present invention as a contrast agent for imaging blood flow in magnetic resonance imaging, preferably in ultra-high-field magnetic resonance imaging operating at static magnetic fields of 7 T or higher.

The last aspect of the invention refers to a method of imaging blood flow in magnetic resonance imaging, preferably in ultra-high-field magnetic resonance imaging operating at static magnetic fields of 7 T or higher, using the nanoparticles of the present invention.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

### BRIEF DESCRIPTION OF THE DRAWINGS

Figure 1. TEM micrographs and corresponding size distribution histograms of DyPO₄ NPs synthesized by solvothermal reaction of butanol solutions of dysprosium nitrate (0.02 M) and different H₃PO₄ concentrations in a microwave oven at 150 º C for 1 h. [H₃PO₄] = 0.075 M (a and b, Dy23 NPs), 0.15 M (c and d, Dy37 NPs), 0.30 M (e and f, Dy57 NPs).
Figure 2: TEM micrographs of DyPO₄ NPs obtained in the experimental conditions used for Dy37 NPs (aging at 150 ºC for 1 hour in a microwave oven a dysprosium nitrate (0.02 M) and H₃PO₄ (0.15M) solution in butanol), changing just one of the parameters as follows: a) Solvent used was octanol, b) Reaction temperature was 120 ºC, c) Reaction temperature was 180 ºC, d) Heating source was a conventional oven.
Figure 3: XRD patterns of Dy23, Dy37 and Dy57 NPs synthesized in the conditions described in Figure 1. Ticks at the bottom of the figure correspond to tetragonal DyPO₄ (Powder Diffraction File of the International Center for Diffraction Data (ICDD) PDF 00-026-0593).
Figure 4: a) DLS size distribution curve in water, b) FTIR spectra and c) thermogravimetry curves of Dy57NPs and Dy57@PAA NPs.
Figure 5: FTIR spectra of Dy23 (a) and Dy37 (b) NPs before and after functionalization with PAA.
Figure 6: Thermogravimetry curves of Dy23 (a) and Dy37 (b) NPs before and after functionalization with PAA.
Figure 7: DLS curves of Dy23 (a) and Dy37 (b) NPs before and after functionalization with PAA.
Figure 8: DLS curves and hydrodynamic sizes of Dy23@PAA, Dy37@PAA, and Dy57@PAA NPs suspended in PBS.
Figure 9: a) Concentration-dependent T₂ weighted phantom image of the Dy23@PAA, Dy37@PAA and Dy57@PAA NPs at 9.4 T. b) T₂ relaxation rates as a function of Dy concentration of Dy23@PAA, Dy37@PAA and Dy57@PAA aqueous suspensions at 9.4 T.
Figure 10: Top: FTIR spectra of the Dy57 NPs functionalized with PAA (Dy57@10PAA) and of the same NPs after coated with PEG (Dy57@10PAA@PEG). The FTIR spectrum of pure PEG is also given with comparative purpose.
Figure 11: Optical microscopy images of HFF-1 fibroblasts resulting from the merge of bright field (grey), DAPI (blue) and TO-PRO-3 Iodine (red) images: a) negative control, b) positive control, c) cells exposed to 100 mM Dy of Dy57@PAA@PEG NPs. Scale bar corresponds to 50 µm. d) Total number of cells per well after exposure to increasing concentration of Dy57@PAA@PEG NPs. e) Percentage of dead cells after exposure to increasing concentration of Dy57@PAA@PEG NPs. e) MTT assay of cells exposed to increasing concentration of Dy57@PAA@PEG NPs
Figure 12. TEM micrograph (a), size distribution histogram (b), X-ray diffraction patterns (c) and DLS curve (d) obtained for the sample synthesized by aging at 120 ºC (in a conventional oven) for 20 h, solutions containing Dy³⁺ (0.02 M) and molybdate (0.2 M) precursors in EG/water (4/1 by vol.) mixtures. The PDF file in (c) corresponds to tetragonal NaY(MoO₄)₂.
Figure 13. TEM micrographs and corresponding particle size distribution histograms obtained for the samples synthesized under the same conditions used for the sample. shown in Figure 1 except: (a and d) Na₂MoO₄/DyCl₃ mole ratio = 5; (b and e) 0.01M DyCl₃ and 0.1M Na₂MoO₄; and (c and f) 0.04M DyCl₃ and 0.4M Na₂MoO₄.
Figure 14. SEM micrograph (a), particle size distribution histogram (b) and DLS curve (c) obtained for the sample synthesized by aging at 120ºC for 20 h in a conventional oven, solutions containing 0.04M DyCl₃, 0.4M Na2MoO4 and 0.04 M tartaric acid.
Figure 15. TEM micrograph (a), size distribution histogram (b), X-ray diffraction patterns (c) and DLS curve (d) obtained for the sample synthesized by aging at 220ºC (in a microwave oven) for 1 h, aqueous solutions containing the Dy³⁺ (0.02 M) and molybdate (0.2 M) precursors and TA (0.04 M). The Powder Diffraction File of the International Center for Diffraction Data (ICDD) PDF file in (c) corresponds to tetragonal NaY(MoO₄)₂.
Figure 16. Top: FTIR spectra of the spherical (Figure 1a) and ellipsoidal particles (Figure 2a); Bottom: TGA curve obtained for the ellipsoidal particles.
Figure 17. SEM micrographs obtained for the samples synthesized under the same conditions used for the sample shown in Figure 2 except: (a) 0.01 M DyCl₃, 0.1 M Na₂MoO₄ and (mole ratio=10) and 0.02 M tartaric acid; (b) 0.06 M DyCl₃, 0.6 M Na₂MoO₄ and (mole ratio=10) and 0.12 M tartaric acid; (c) 0,02 M DyCl₃ y 0,1 M Na₂MoO₄ and (mole ratio =5); and (d) 0,02 M DyCl₃ y 0,4 M Na₂MoO₄ and (mole ratio =20).
Figure 18. SEM micrographs (left) and corresponding histograms (right) obtained for the samples synthesized by keeping the same experimental conditions involved in the synthesis of the sample shown in figure 2 but using water-EG mixtures as solvent with different volumetric water/EG ratios: 6/4 (a), 5/5 (b) and 4/6 (c).
Figure 19. TEM micrographs (left) and corresponding histograms (right) obtained for the samples synthesized by keeping the same experimental conditions involved in the synthesis of the sample shown in Figure 4c but using different amounts of TA: 0.02 M (a) and 0.06 M (b).
Figure 20. FTIR spectra (top) and TGA curves (bottom) obtained for ellipsoidal NPs having different size.
Figure 21. DLS curves (top) and X-ray diffraction patterns (bottom) obtained for the ellipsoidal NPs having different size. The PDF file corresponding to tetragonal NaY(MoO₄)₂ is included.
Figure 22. T₂-weighted phantom images (top) and T₂ relaxation rates as a function of Dy concentration (bottom) obtained for aqueous suspensions of the spherical and ellipsoidal NaDy(MoO₄)₂ NPs with different sizes.
Figure 23. DLS curves obtained for uncoated and coated NaDy(MoO₄)₂ spheres and ellipsoids dispersed in water and PBS buffer.
Figure 24. FTIR spectra obtained for the coated samples: Spheres 23 nm and Ellipsoids 50 nm.
Figure 25. T2 relaxation rates as a function of Dy concentration obtained for aqueous suspensions of the coated NaDy(MoO₄)₂ nanospheres and ellipsoids.
Figure 26. Optical microscopy images of HFF-1 fibroblasts resulting from the merge of bright field (grey), DAPI (blue), and TO-PRO-3 Iodine (red) images: negative control (a); positive control (b); cells exposed to 100 mM Dy of Spheres 23 nm (c) and cells exposed to 100 mM Dy of Ellipsoids 50 nm (d). Scale bar corresponds to 100 mm.
Figure 27. Total number of cells per well (left column), percentage of dead cells (middle column), and MTT assay of cells (right column) after exposure to increasing concentration of spherical (23 nm) (a, b, c), ellipsoidal (50 nm) (d, e, f) NPs.
Figure 28. Cell uptake for coated NaDy(MoO₄)₂ nanoparticles with spherical (sample Spheres 23 nm) and ellipsoidal (sample Ellipsoids 50 nm) shape in HFF-1 fibroblasts after 1 and 24 h of exposure determined by ICP-HRMS.

### Examples

### Example 1 DyPO₄ nanoparticles

*Reagents:* Dysprosium nitrate hydrate (Dy(NO₃)₃·5H₂O, Sigma Aldrich, 99.99%), phosphoric acid (Sigma Aldrich, 85%), butanol (Sigma Aldrich, >99.5%), and 1-octanol (Merck, >99%) were used for NPs synthesis. Poly(acrylic acid) (PAA, average Mw ~ 1800, Sigma-Aldrich) was used for NPs functionalization with PAA. For NPs functionalization with PEG, the following reagents were used as received: 1-(3-Dimethylaminopropyl)-3-ethyl carbodiimide (EDC - Sigma Aldrich, USA), N-Hydroxysulfosuccinimide (s-NHS -Sigma Aldrich, USA) and α-Methoxy-ω-amino PEG of 5000 Da (PEG, RappPolymer, Germany).

Colloidal stability of NPs was evaluated using an aqueous solution of Phosphate Buffered Saline (PBS, Sigma Aldrich) that was prepared as follows: one tablet of PBS was dissolved in 200 mL water to obtain 137 mM NaCl, 2.7 mM KCI and 10 mM phosphate buffer, pH 7.4 at 25 ºC). An aqueous solution of sodium tetraborate decahydrate (Sigma Aldrich, > 99.5 %) 10 mM was prepared and its pH was adjusted to 9 with NaOH. We call this solution borate buffer from now on.

*Synthesis:* For the synthesis of uniform dysprosium phosphate nanoparticles, we used a homogeneous precipitation reaction. Essentially, the method consists of aging a mixture of Dy(NO₃)₃ with phosphoric acid in butanol at 150 ºC in a microwave oven. The standard procedure is as follows: Dysprosium nitrate was dissolved in 30 mL of butanol at 50 ºC for 1 h under magnetic stirring to facilitate dissolution in order to get a 0.02 M dysprosium nitrate solution. Once dissolved, the solution was admixed with 616 microliters of H₃PO₄ to obtain a final solution with 0.30 M H₃PO₄ concentration. The mixture was aged in tightly closed test tubes using a microwave oven at 150 ºC for 1 h. The resulting dispersion was cooled down to RT, centrifuged to remove the supernatants, and washed, twice with ethanol and once with double distilled water.

The NPs were finally stored either in distilled water or dried at 50 °C for some analyses.

Additional experiments were conducted to analyze the effect of synthesis parameters (solvent nature, H₃PO₄ concentration, aging temperature, and heating source) on the characteristics of the precipitated nanoparticles.

*Functionalization with PAA:* The aqueous suspension of NPs obtained as described above was diluted in Milli-Q water to get a concentration of 1 mg/mL and its pH was adjusted to 10 with 1M NaOH solution. PAA (2 mg/mL) was then added to the suspension and, again, the pH was adjusted to 10. The resulting suspension was magnetically stirred for 1 h at RT and centrifuged at 14000 rpm for 20 min to remove the supernatant. The resulting NPs were washed twice with Milli-Q water through centrifugation in the same conditions and stored in Milli-Q water. The pH of the resultant suspension was close to 7.

*Further Functionalization with PEG:* The NPs showing the optimum magnetic relaxivity value in this study were further functionalized with PEG. For PEG coating, the NPs were firstly functionalized with PAA following the same procedure described above but using 10 mg/mL PAA to increase the number of carboxylate groups on the NPs surface for PEG grafting. The obtained NPs were then suspended in borate buffer (6 mg NPs/ml). A volume of 0.94 ml of an EDC solution in borate buffer (3.2 mg/ml) was admixed with 0.85 ml of s-NHS solution in borate buffer (6 mg/ml). The mixture was shaken at RT for 10 min in the end-over-end roller. A volume of 161 µl of the NPs@10PAA suspension was added to the resulting solution. The three mentioned volumes were chosen in order to get the following molar ratio: 1 COOH:10 EDC:15 NHS (COOH refers to the carboxylic groups in PAA attached to the NPs). The resultant suspension was stirred at RT for 2h in the end-over-end roller and admixed with 1.56 ml of a PEG solution in borate buffer (10 mg/ml) (in accordance with the ratio 1 COOH:2 PEG to block the unreacted COOH groups and to increase the colloidal stability of the NPs). Finally, after 45 minutes of stirring at RT in the end-over-end roller, NPs were separated by centrifugation at 14500 rpm for 5 minutes. The resulting NPs were washed twice with borate buffer and they were suspended in 1 mL of fresh borate buffer and stored at 4°C. This protocol was repeated ten times to have enough NPs for the rest of the analyses.

### Chemical stability studies

NPs functionalized with PAA were suspended in PBS (pH= 7.4) and the suspension was magnetically stirred at 37 ºC for several weeks. A portion of the suspension was extracted and centrifuged after 1, 3, and 5 weeks and the supernatants were analyzed by ICP to determine the fraction of dissolved dysprosium.

### Characterization techniques

Transmission electron microscopy (TEM, JEOL 2100Plus) was used to determine particles shape and size. To prepare the sample for the TEM analyses, a droplet of the NPs aqueous suspension was deposited on a copper grid coated with a transparent polymer. Particle size distribution curves were obtained by counting about one hundred of particles on the TEM micrographs using the ImageJ open-source image processing program.

The colloidal stability of the NPs before and after functionalization was assessed by Dynamic Light Scattering (DLS, Malvern Zetasizer Nano-ZS90). DLS curves were obtained both in aqueous and PBS suspensions with ~ 0.5 mg NPs/ml.

Ion Coupling Plasma (ICP) analyses were carried out in an iCAP 7200 ICP-OES Duo (ThermoFisher Scientific) apparatus.

X-ray powder diffraction (XRD) was used for phase identification. The XRD patterns were recorded with a Panalytical X'Pert Pro diffractometer (Cu Kα) with an X-Celerator detector in the angular range of 10 º < 2θ < 80 ° using a 2θ step width of 0.02 ° and 10 s counting time. The crystallite size was estimated using the Scherrer formula from the full width at half maximum (FWHM) of a single reflection. A shape factor of k = 0.9 and an instrumental broadening factor of 0.112 were used for the calculation.

The success of the functionalization processes was proved using Fourier Transform Infrared (FTIR) spectroscopy and Thermogravimetric analyses (TGA). FTIR (Jasco FT/IR-6200) was conducted on NPs pellets diluted in KBr while TGA curves were recorded in a Q600TA Instrument at a heating rate of 10°C·min-1 in air.

### Results

### a. NPs synthesis, morphology and crystal phase.

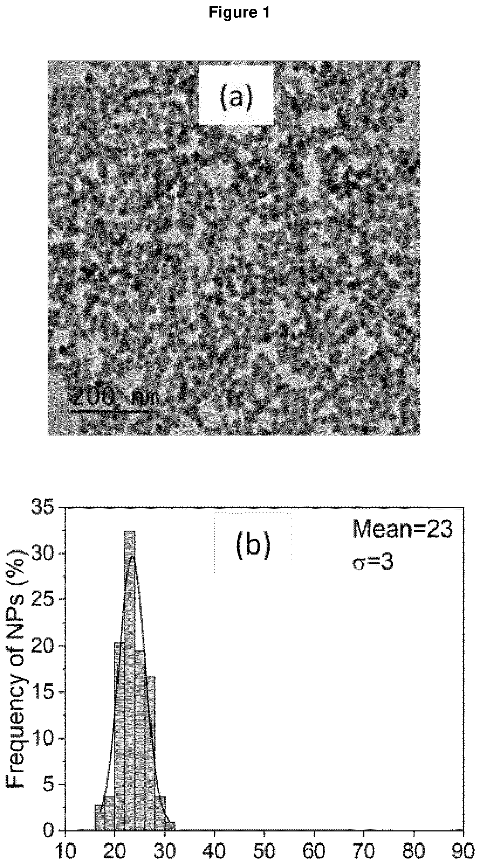

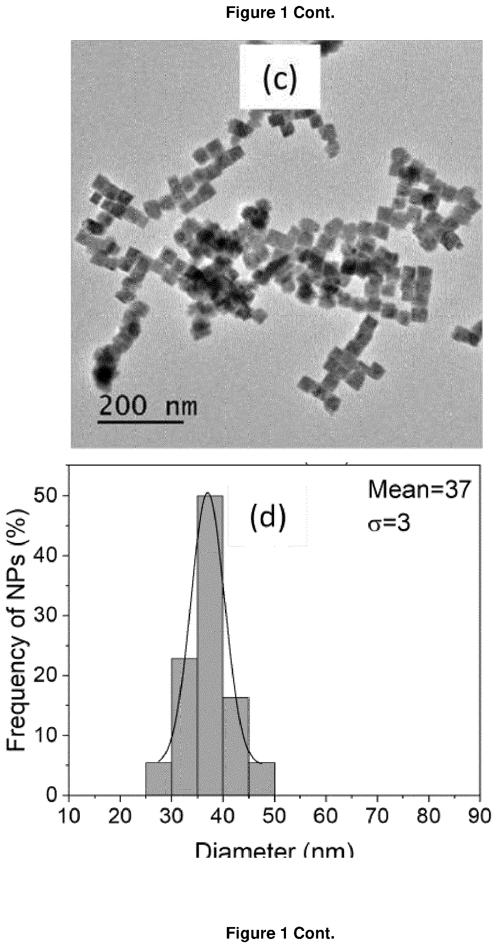

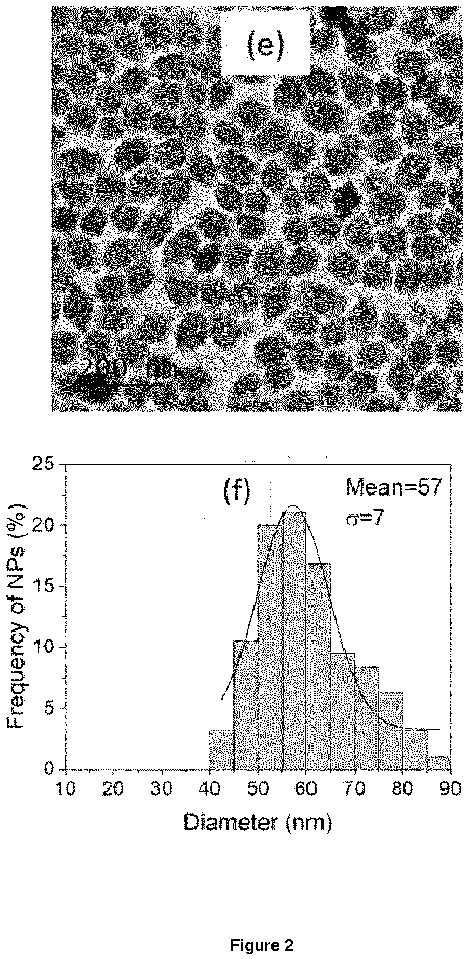

TEM micrographs in Figure 1 show the NPs obtained after aging, at 150 ºC for 1 hour in a microwave oven (MW), three butanol solutions containing dysprosium nitrate (0.02 M) and phosphoric acid (0.075 M, 0.15 M, and 0.30 M). All three micrographs showed uniform NPs whose size increased with increasing H₃PO₄ concentration, as observed in the histograms plotted in the same Figure 1. Specifically, the use of very dilute (0.075 M) H₃PO₄ resulted in cube-like NPs with an average value of the face diagonal of 23 nm, while doubling H₃PO₄ concentration to 0.15 M gave also rise to cube-like NPs showing a larger face diagonal size (37 nm). Finally, when the concentration of H₃PO₄ was increased to 0.30 M, more elongated NPs were precipitated with a long axis length of 57 nm. The H₃PO₄ concentration allowed therefore tuning the NPs size from 23 nm to 57 nm while keeping their uniform shape. The NPs shown in Figure 1 will from now on be called Dy23, Dy37 and Dy57, in reference to their size.

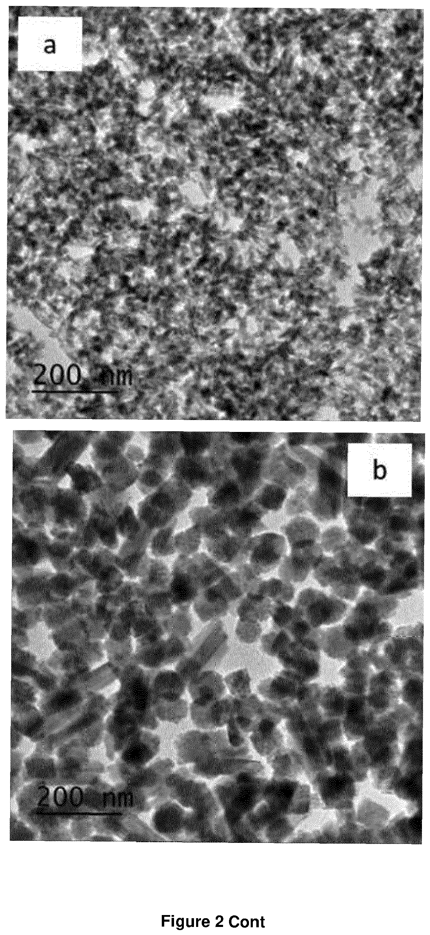

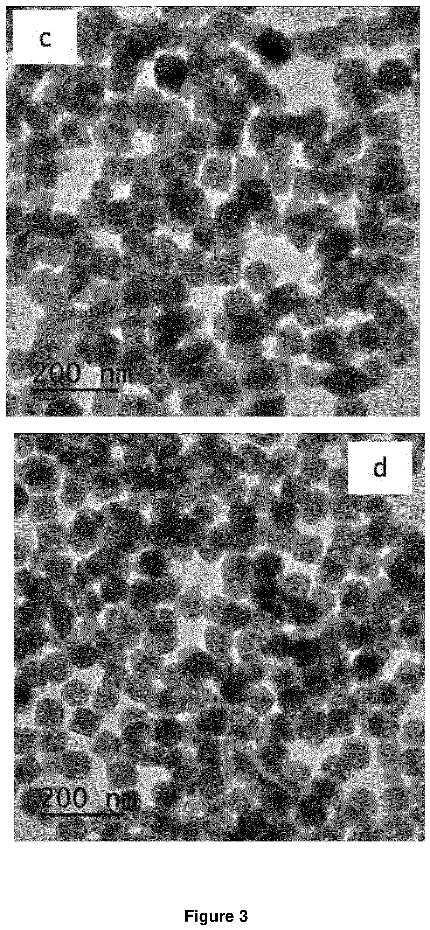

The effect of other experimental parameters such as solvent nature, aging temperature, and heating source on the NPs morphology was subsequently analyzed. Figure 2 shows the TEM micrographs of the precipitates obtained by changing just one of the experimental conditions used to synthesize the NPs shown in Figure 1c (Dy37). It can be observed that the solvent nature produced a drastic change in the precipitate obtained, so that using octanol instead of butanol resulted in the precipitation of heterogeneous, fully aggregated tiny NPs (Figure 2a).

Another key factor for the synthesis of uniform NPs was the reaction temperature. When the reaction was carried out at 120 ºC (Figure 2b) the coexistence of both, heterogeneous rods and cubic-like NPs was observed in the reaction product while increasing the temperature to 180 ºC gave rise to uniform, cubic-like NPs with -70 nm edge size (Figure 2c). A minimum reaction temperature of 150 ºC was, therefore, necessary to obtain homogeneous NPs, while increasing the temperature to 180 ºC gave rise to an increase in particle size.

Finally, when conventional heating was used instead of MW heating, aggregated, cubic-like NPs with -65 nm edge size were observed (Figure 2d). Conventional heating resulted, therefore, in the precipitation of larger particles compared to MW heating. All these changes must be assigned to variations in the kinetics of precipitation which also affect the nucleation and growth processes.

The crystal phase of the Dy23, Dy37, and Dy57 NPs was analyzed by XRD (Figure 3). All three patterns exhibited the same set of reflections, compatible with tetragonal DyPO₄ (Powder Diffraction File of the International Center for Diffraction Data (ICDD) PDF 00-026-0593). No additional reflections were observed in any of the patterns, indicating the absence of other crystalline phases. It can be noted that the width of the reflections decreased with increasing particle size, indicating that the crystallite size increased correspondingly. The crystallite size was estimated from the half-width at half maximum of the reflection located at -35 °2θ using the Scherrer formula. Crystallite size values of 26 nm, 34 nm, and 52 nm were obtained for the Dy23, Dy37, and Dy57 NPs, respectively. The value of the crystallite size, very close to the NPs size in each case, suggesting that the NPs were single crystal in nature.

### b. Analysis of the colloidal and chemical stability.

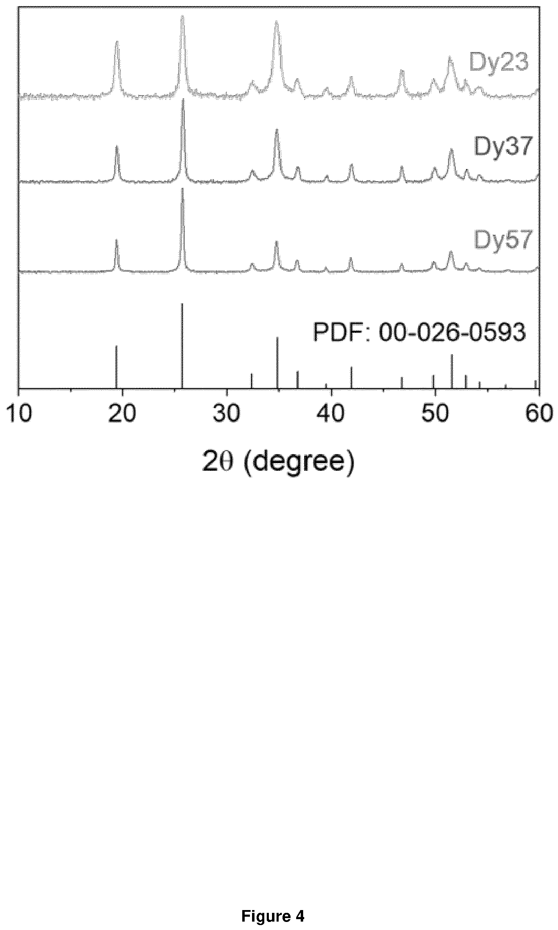

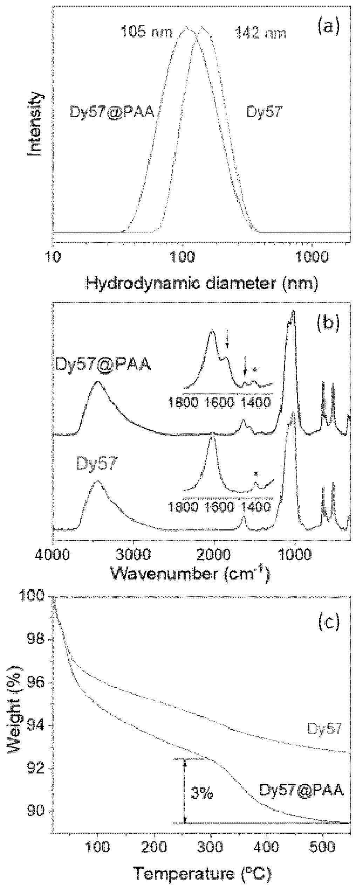

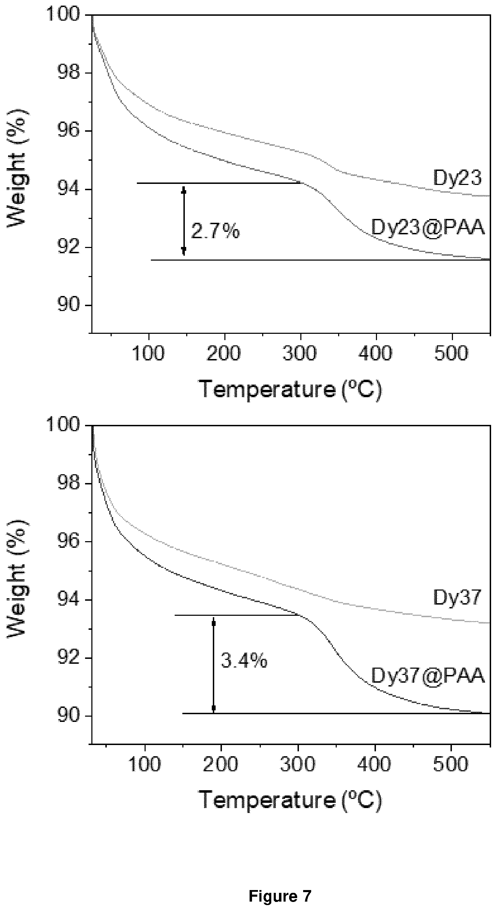

The colloidal stability of Dy23, Dy37 and Dy57 NPs suspended in water was evaluated from DLS curves (Figure 4a and Figure 7). Mean hydrodynamic diameters of 240 nm, 164 nm, and 142 nm, respectively, were obtained for Dy23, Dy37, and Dy57. These values, higher than the dimensions of the particles obtained from TEM micrographs, indicated that all three types of NPs were aggregated in water.

Functionalization of the NPs with PAA was carried out to try to reduce such aggregation. The NPs obtained after such functionalization process will be called Dy23@PAA, Dy37@PAA and Dy57@PAA from now on. The success of the functionalization process was monitored through FTIR spectroscopy and thermogravimetry curves.

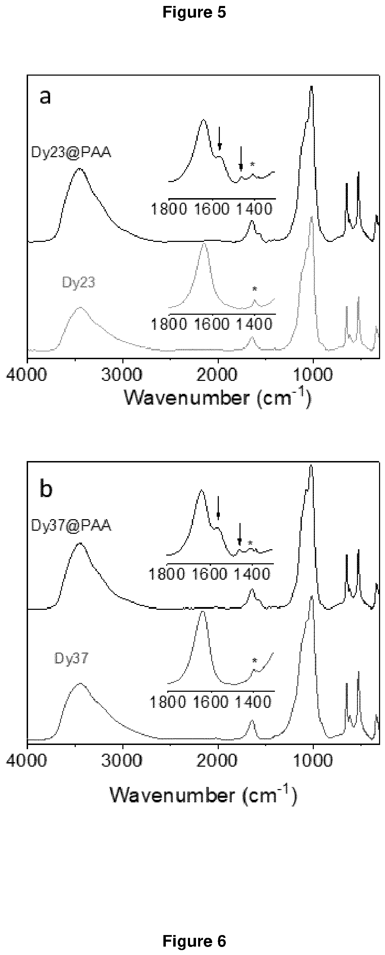

Figure 4b shows the FTIR spectra of Dy57 NPs before and after PAA coating. The corresponding FTIR spectra of Dy23 and Dy37 NPs are plotted in Figure 5. The spectra of the three NPs before functionalization showed the characteristic bands associated with the phosphate vibrational modes (located at wavenumbers < 1250 cm⁻¹) and adsorbed water (at 3400 cm⁻¹ and 1625 cm⁻¹) plus a low-intensity band at around 1400 cm⁻¹ (marked with an asterisk) that can be assigned to the presence of residual solvent molecules on the NPs surface.

On the other hand, the FTIR spectra of all three NPs after functionalization showed two additional bands at ~ 1460 cm⁻¹ and 1560 cm⁻¹ (labelled with arrows) that can be assigned to the vibrations of the carboxylate groups of the PAA molecules.

On the other hand, the TG curve of Dy57 NPs before PAA functionalization (Figure 4c) showed a first weight loss of around 4 % at T < 100 ºC, corresponding to the removal of adsorbed water and a second, progressive one at T > 100 ºC that could be assigned to the decomposition of the residual solvent molecules. The curve corresponding to the Dy57@PAA NPs showed, in addition to both weigh losses, a steeper loss of around 4 % at ~300 ºC that can be due to the decomposition of PAA entities.

Similar TG curves were observed for Dy23 and Dy37 before and after functionalization (Figure 6). The FTIR and TG results demonstrate, therefore, that all three types of NPs were successfully coated with PAA.

The colloidal stability of the functionalized NPs suspended in water was then reevaluated using DLS. The DLS curves of the functionalized NPs significantly shifted towards lower hydrodynamic values with respect to those recorded before functionalization, as observed in Figure 4a for Dy57@PAA NPs and in Figure 7 for Dy23@PAA and Dy37@PAA. It can be therefore concluded that the functionalization process notably improved the colloidal stability of the NPs in water.

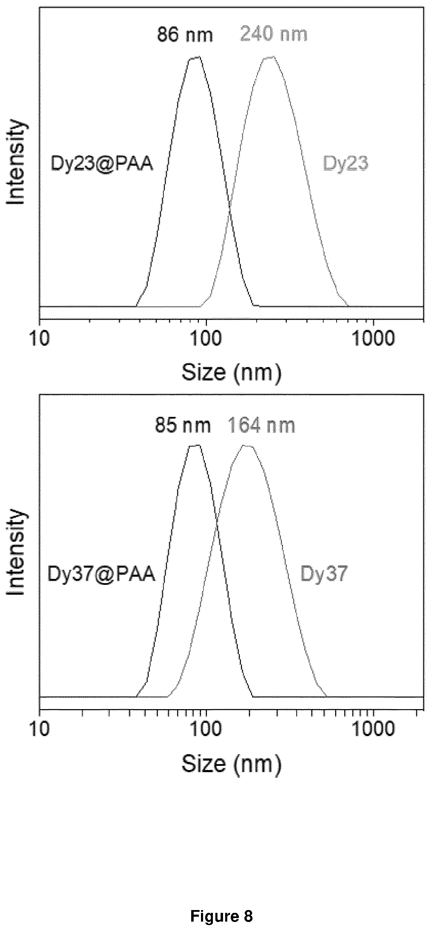

Finally, the PAA-functionalized NPs were suspended in PBS (pH= 7.4) to assess their colloidal stability in a buffer solution commonly used in biological research to simulate physiological fluid. All three NPs functionalized with PAA could be easily re-dispersed in the buffer, as demonstrated by the DLS curves shown in Figure 8, with hydrodynamic sizes between 80 nm and 100 nm.

It is well known that, in addition to their colloidal stability in physiological fluid, materials intended to be applied in nanomedicine should be chemically stable, i.e., nanoparticles dissolution should not take place in the physiological fluid during the period between the injection and the excretion of the NPs to hinder potential toxic ions to be released to the blood stream. As a proof of concept, we have studied the chemical stability at 37 ºC of the Dy57@PAA NPs suspended in PBS (pH= 7.4) with time. The value of Dy fraction dissolved after 1-, 3-, and 5-weeks incubation was 0.15 %, 0.10 %, and 0.72 %, respectively, which indicates a negligible degree of dissolution of DyPO₄ in PBS. This value was, in addition, significantly lower than those reported for fluoride-based NPs (NaYF₄ and KYF₄) incubated in the same conditions for just 3 days, which showed between 30 % - 45 %, respectively, of fluor dissolved. In conclusion, DyPO₄@PAA NPs show an excellent degree of chemical stability in PBS.

### c. Relaxivity study

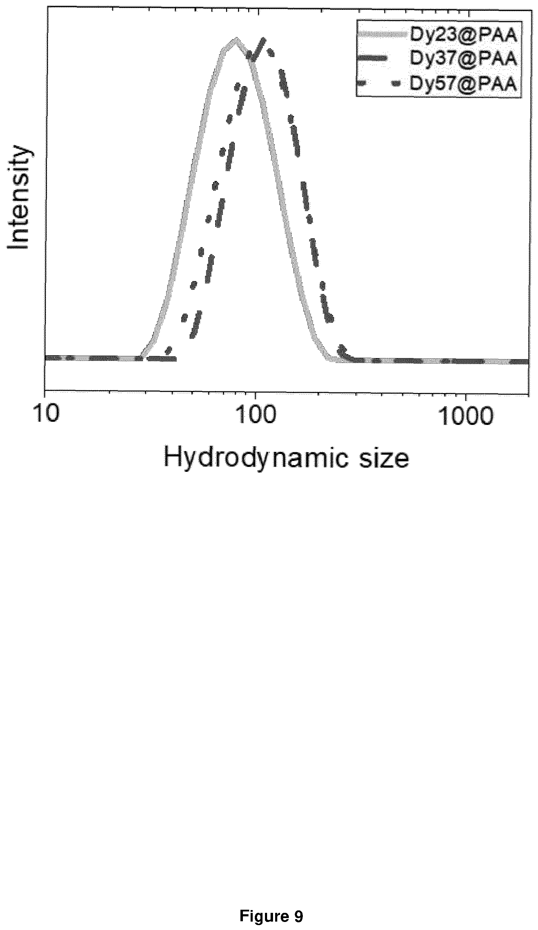

The performance of the three different NPs@PAA as UHF MRI contrast agents was evaluated using a preclinical 9.4 T MRI scanner at room temperature (RT, 23°C). Figure 9a shows the T₂-weighted MR images obtained in aqueous suspensions with different concentrations of Dy23@PAA, Dy37@PAA, and Dy57@PAA NPs. The images became darker as the NPs concentration increased for all three samples, and they turned practically black at concentrations as low as ~ 0.3 mM, which indicates that the NPs behaved as good negative MRI contrast agents.

The inverse of the experimentally determined transverse relaxation rates (1/T₂) of water protons was plotted against the molar concentration of Dy³⁺ in the aqueous suspensions (Figure 9b). The r₂ values, determined from the slope of the fitted line, were as high as 395 mM⁻¹s⁻¹, 432 mM⁻¹s⁻¹, and 516 mM⁻¹s⁻¹ for Dy23@PAA, Dy37@PAA, and Dy57@PAA NPs, respectively. The r₂ values increased, therefore, with increasing particle size.

This trend agrees well with the literature for other nanoparticulated systems such as HoF₃(Baek, A.; Kim, H. K.; Yang, J. U.; Choi, G.; Kim, M.; Cho, A. E.; Kim, Y. H.; Kim, S.; Sung, B.; Yang, B. W.; et al. High-performance hepatobiliary dysprosium contrast agent for ultra-high-field magnetic resonance imaging. Journal of Industrial and Engineering Chemistry 2020, 85, 297-307) NaHoF₄ [Ni, D. L.; Zhang, J. W.; Bu, W. B.; Zhang, C.; Yao, Z. W.; Xing, H. Y.; Wang, J.; Duan, F.; Liu, Y. Y.; Fan, W. P.; et al. PEGylated NaHoF4 nanoparticles as contrast agents for both X-ray computed tomography and ultra-high field magnetic resonance imaging. Biomaterials 2016, 76, 218-225], NaDyF₄ [Gonzalez-Mancebo, D.; Becerro, A. I.; Rojas, T. C.; Garcia-Martin, M. L.; de la Fuente, J. M.; Ocana, M. HoF3 and DyF3 Nanoparticles as Contrast Agents for High-Field Magnetic Resonance Imaging. Particle & Particle Systems Characterization 2017, 34 (10*)],* Dy₂O₃ [Das, G. K.; Johnson, N. J. J.; Cramen, J.; Blasiak, B.; Latta, P.; Tomanek, B.; van Veggel, F. NaDyF4 Nanoparticles as T-2 Contrast Agents for Ultrahigh Field Magnetic Resonance Imaging. Journal of Physical Chemistry Letters 2012, 3 (4), 524-529], and HoPO₄ [Becerro, A. I.; Ocana, M. Quick synthesis, functionalization and properties of uniform, luminescent LuPO4-based nanoparticles. Rsc Advances 2015, 5 (44), 34517-34524*],* where an increase in transversal relaxivity was observed with increasing nanoparticle size. This effect is due to the dependence of the proton relaxation enhancement on spins correlation times. The relaxivity values reported for our DyPO₄@PAA NPs were, to the best of our knowledge, the highest r₂ values ever reported at 9.4 T for any Dy-based nanometric particles in the literature, such as DyF₃, NaDyF₄, or Dy₂O₃, which showed r₂ values between 100 mM⁻¹s⁻¹ and 380 mM⁻¹s⁻¹.

Sample Dy57@PAA, showing the highest r₂ value (516 mM⁻¹s⁻¹) among the three NPs of this study, was subsequently submitted to the analysis of colloidal stability in physiological media and it was used as well for *in vivo* experiments.

### d. Biocompatibility studies

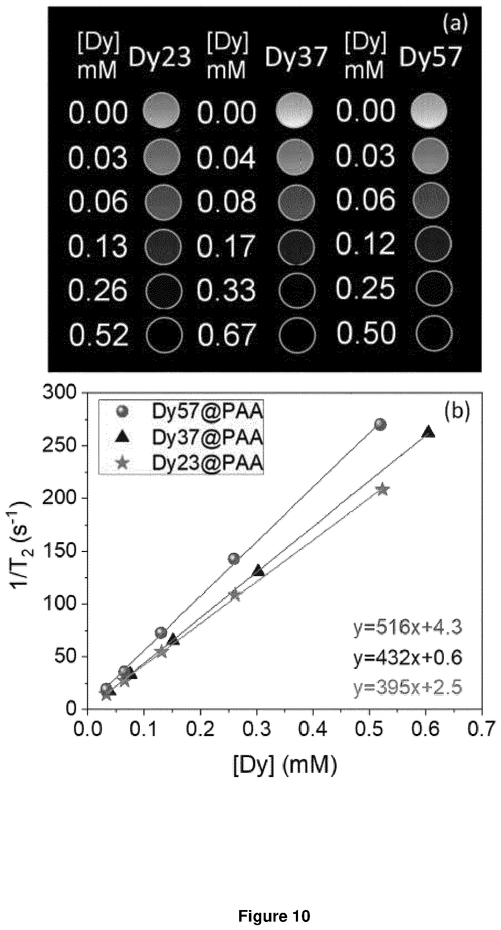

Previous to biocompatibility studies, the Dy57@PAA NPs were coated with PEG, with the purpose of improving their systemic circulation time and avoiding their clearance by the immune system. The NPs were successfully PEGylated, as demonstrated by the FTIR spectra shown in Figure 10. The same figure also contains the DLS curve of the corresponding NPs suspended in PBS, showing a hydrodynamic size of 164 nm. The PEGylated sample was submitted as well to magnetic resonance studies, obtaining a value for r₂ of 504 mM⁻¹·s⁻¹, very close to that of the NPs before PEGylation (516 mM⁻¹·s⁻¹), which indicates a negligible effect of the PEG shell on the relaxivity capacity.

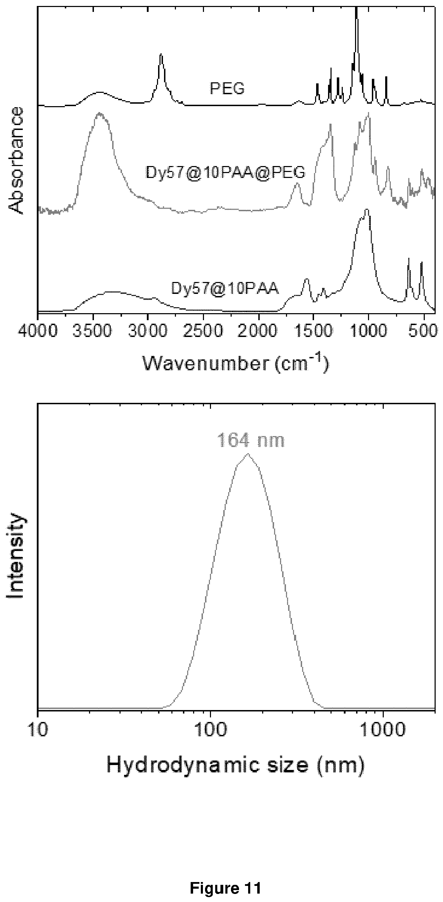

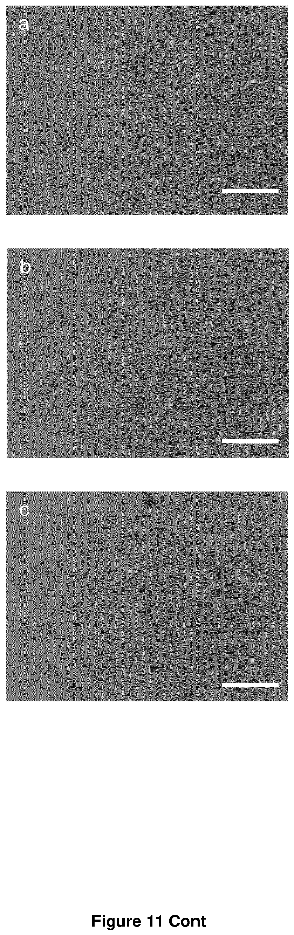

Biosafety studies of Dy57@PAA@PEG NPs were evaluated in HFF-1 human foreskin fibroblasts. Several parameters were examined to get a complete assessment of the cytotoxic behaviour, such as cell morphology, induction of necrotic/late apoptotic cells, and mitochondrial activity. When merging brightfield, DNA staining (DAPI or Hoechst 33342), and nuclei staining (TO-PRO-3 Iodine) to get microscopy images, it was found that cells exposed to a NPs concentration up to 100 mM (referred to Dy³⁺), did not show any appreciable morphological change (Figure 11a-c).

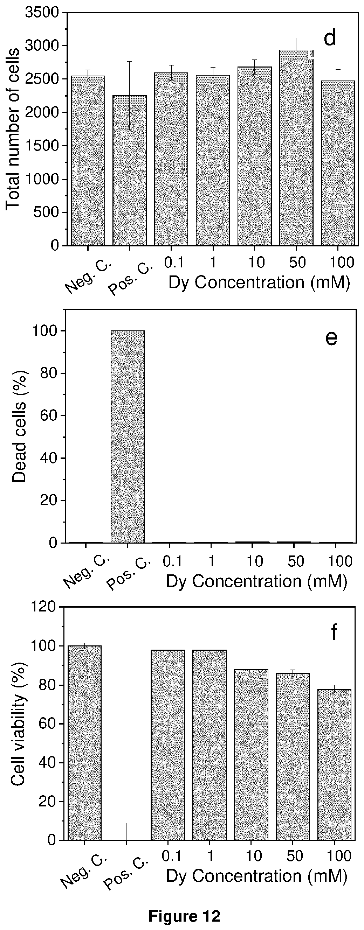

On the other hand, cellular necrosis and apoptosis were assessed in the "live-dead" assay. The total number of cells per well remained unaltered for all the NPs concentrations tested (Figure 11d) while these NP concentrations did not give rise to a relevant increase in cell death, which remained very close to zero in all cases (Figure 11e).

Finally, no statistically significant effect on mitochondrial activity was observed with the MTT assay, which showed a cell survival close to 100 % for any NPs concentration up to 100 mM Dy (Figure 11f).

These results clearly indicate negligible toxicity effects under the assayed conditions, and, therefore, the suitability of the DyPO4@PAA@PEG NPs as bioimaging probes.

### Example 2 Spherical and ellipsoidal NaDy(MoO₄)₂ particles

*Reagents:* Dysprosium (III) chloride hexahydrate (DyCl₃. 6H₂O, Sigma Aldrich, 99.9%), sodium molybdate (Na₂MoO₄, Sigma Aldrich, ≥ 98 %), ethylene glycol (EG, Sigma Aldrich, 99.8 %), (2R, 3R)-(+)-Tartaric acid (TA, C₄H₆O₆, Sigma Aldrich), poly(allylamine hydrochloride) (PAH, average Mw ~ 17500 Da), and polyacrylic acid (PAA, Sigma Aldrich, Mw 1800) were used as received. Phosphate buffered saline (PBS) consisting of a 137 mM NaCl, 2.7 mM KCI and 10 mM phosphate solution with pH = 7.4 at 25 ºC, was prepared by dissolving a PBS tablet (Sigma-Aldrich) in 200 mL Milli-Q water.

*Synthesis:* The spherical NaDy(MoO₄)₂ NPs were prepared as follows. Sodium molybdate was dissolved in 1 mL Milli-Q water and after that, 1.5 mL of EG was added. In another vial, dysprosium chloride was dissolved in 2.5 mL of EG at -80 °C under magnetic stirring. Once the latter was cooled down to room temperature, both solutions were admixed. The concentrations of Dy³⁺ and MoO₄²⁻ in the final solution were 0.02 M and 0.2 M, respectively. After the mixture was homogenized, it was aged for 20 hours in a conventional oven preheated at 120 ºC. The total concentration of precursors in the starting solutions and the Dy/Mo molar ratio were modified aiming to modulate the size of the precipitated particles.

For the synthesis of the ellipsoidal NPs, a novel procedure was developed. It essentially consisted of a homogeneous precipitation reaction from Dy³⁺ and MoO₄²⁻containing solutions in different solvents and in the presence of tartaric acid used as a Dy³⁺ complexing agent. The standard procedure in the case of using water as solvent was as follows. Weighted amounts of sodium molybdate, dysprosium chloride, and TA were separately dissolved in 5 mL, 2.5 mL, and 2.5 mL of Milli-Q water, respectively. These solutions were admixed so that the final concentrations of Dy³⁺, MoO₄²⁻ and TA were 0.02 M, 0.2 M, and 0.04 M, respectively. After the mixture was homogenized, it was aged for 30 minutes at 220 ºC in a microwave-assisted oven (Anton Paar Monowave 300). In order to modify the size of the precipitated ellipsoidal particles, the solvent nature was modified by the addition of different quantities EG to the water solutions, and the total concentration of precursors, the Dy/Mo molar ratio, and the TA concentration were systematically varied.

In all cases, the precipitates obtained after aging were cooled down to room temperature. Then, they were centrifuged to remove the supernatants and washed, twice with ethanol and once with double distilled water. The obtained particles were finally stored in Milli-Q water and, for some analyses, they were dried at 50 ºC.

*Functionalization:* Polyelectrolytes of opposite electrical charge were adsorbed by electrostatic interactions on the surface of the nanoparticles in a sequential and alternating way. PAH was used as the positively charged polyelectrolyte and PAA as the negatively charged one. The first positive PAH layer was formed/deposited as follows: 5 mL of an 0.05 M NaCl aqueous solution at pH 6.5 containing 10 mg mL⁻¹ of PAH were added to 1.5 mL of a water dispersion containing 5 mg of the NPs. The resulting mixture was first sonicated in iced water for 5 minutes and then kept for 15 minutes at room temperature in an end-over-end shaker. Afterward, the dispersion was centrifuged to remove the supernatant and the NPs washed twice with Milli-Q water. For the forming/deposition of the second PAA negative layer on the spherical NPs, a similar procedure was followed, except that PAH was substituted by PAA. The resulting functionalized NPs were dispersed and stored in Milli-Q water.

### Chemical stability

The chemical stability of the samples was studied by aging a NPs dispersion (3 mg mL⁻¹) in PBS buffer at 37 ºC to mimic the physiological conditions. After aging, the dispersion was centrifuged and the supernatants analyzed by ICP.

### Characterization techniques

Transmission electron (TEM) and scanning electron (SEM) micrographs were obtained using a JEOL 2100Plus (200 kV) or a Hitachi S4800 microscope, respectively. The ImageJ software was employed to obtain the size distribution histograms from the micrographs.

X-ray diffraction (XRD) patterns were acquired using a Panalytical, X'Pert Pro diffractometer (CuKα) with an X-Celerator detector over an angular range (2θ) from 10 ° to 90 °, 2θ step width of 0.03 °, and 10 s counting time.

The infrared spectra (FTIR) of the NPs dispersed in KBr pellets were recorded in a Jasco FT/IR-6200 Fourier transform spectrometer. Thermogravimetric analyses (TGA) were conducted in an air atmosphere at a heating rate of 10 °C min⁻¹, using a TA Instruments-TGA Discovery apparatus.

The hydrodynamic diameter of the NPs in aqueous or PBS suspensions (0.5 mg·mL⁻¹) before and after functionalization was analyzed by Dynamic Light Scattering (DLS) using a Malvern Zetasizer Nano-ZS90 equipment. This instrument was also used to measure Zeta potential.

ICP analyses were carried out in an iCAP 7200 ICP-OES Duo (ThermoFisher Scientific) apparatus.

Transverse magnetic relaxivity (r₂) was determined at 9.4 T using a Bruker Biospec MRI system. T₂ values were determined in aqueous suspensions of NPs with different concentrations of Dy, ranging from 0.03 mM to 0.5 mM, using the Carl-Purcell-Meiboom-Gill (CPMG) sequence. Then, r₂ values were obtained from the slope of the linear fit of 1/T₂ vs. the concentration of Dy expressed in mM.

Cytotoxicity was assessed in HFF-1 cells by Live-Dead and MTT assays (Journal of Colloid and Interface Science 2021, 587, 131-140. Briefly, the "live-dead assay" was as follows: The HFF-1 cells were cultured with a medium containing the Dy nanoparticles in varying concentrations. After 24 h, Triton X-100 was added to the positive control wells. After 15 min, all the wells were stained with DAPI (4',6-Diamidino-2-phenylindole) to label nuclei in all cells, although with stronger labeling in live cells, and TO-PRO-3 Iodine to only label dead cells. The cell morphology images were acquired using a Perkin Elmer Operetta High Content Imaging System. For each field, fluorescence images for DAPI and TO-PRO-3, plus a brightfield image were captured. Cell mortality percentages were calculated automatically by Operetta Harmony software, whereby all nuclei (dead and alive) were identified from the DAPI staining and the percentage of dead cells then determined by the number of nuclei also possessing high levels of TO-PRO-3 staining. For the "MTT assay" the HFF-1 cells were cultured with medium containing the Dy nanoparticles in varying concentrations. After 24 h, the supernatant was replaced by fresh medium containing 3-[4,5-dimethylthiazol-2-yl]-2,5-diphenyl tetrazolium bromide (MTT). After 2 h of incubation the medium was removed and the formazan crystals were solubilized with DMSO. The absorbance of each well was read on a microplate reader (Dynatech MR7000 instruments) at 550 nm. The relative cell viability (%) and its error related to control wells containing cell culture medium without nanoparticles were calculated by the equations given in Journal of Colloid and Interface Science 2021, 587, 131-140.

Cellular uptake was determined quantitatively by Inductively Coupled Plasma High-Resolution Mass Spectroscopy (ICP-HRMS, Perkin-Elmer NexION) in HFF-1 cells as described previously by some of us. Briefly, cells were plated at a density of 1 x 10⁴ cells per well in a 96-well plate. After 24 h, the medium in the wells was replaced with 0.1 mL of a fresh medium containing NPs at a concentration of 100 µg mL⁻¹ (total amount of NPs = 10 µg), which corresponds to 0.2 mM Dy. The uptake was evaluated at 1 h and 24 h. Once this time had elapsed, wells were washed with PBS, and 200 µL of aqua regia were added, recovering the liquid after 8 h and diluting it with 19.8 mL of water.

### Results

### a Nanoparticles synthesis and characterization

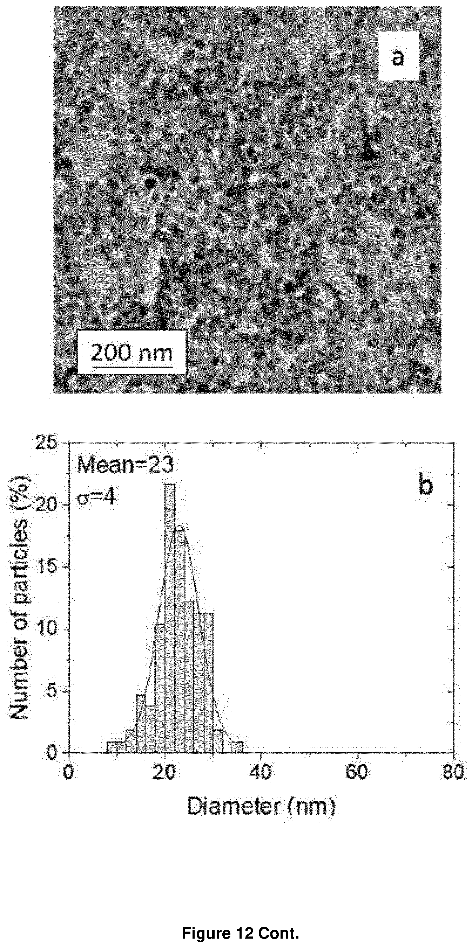

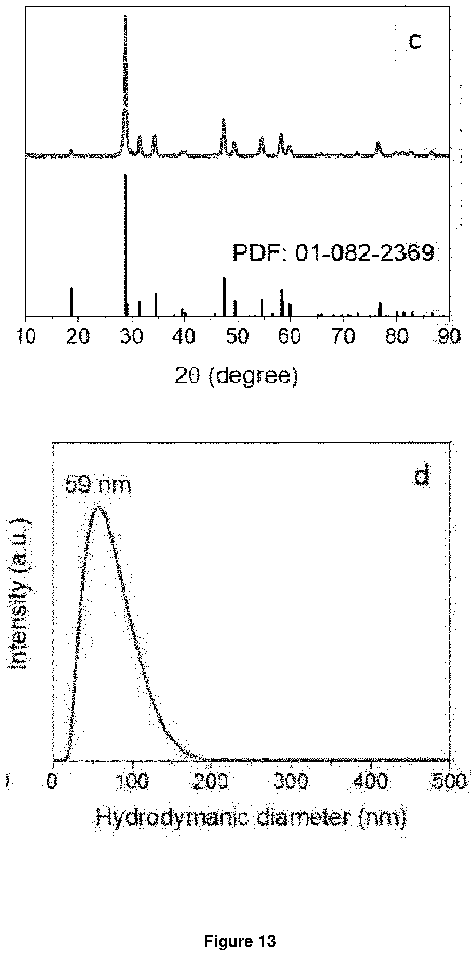

The method previously developed by us for the synthesis of NaY(MoO₄)₂ nanospheres consisting of aging at 120 º C (in a conventional oven) for 20 h, solutions containing Y³⁺ (0.02 M) and molybdate (0.2 M) precursors in EG/water (4/1 by vol.) mixtures, was found to be successful when adapted to the NaDy(MoO₄)₂ system by replacing Y³⁺ with Dy³⁺. Thus, almost spherical particles (Figure 12a) having a mean diameter of 23 nm and a rather narrow size distribution (standard deviation, α = 4) (Figure 12b) were obtained, which consisted of tetragonal NaDy(MoO₄)₂ as indicated by their XRD pattern, which was quite similar to that reported for tetragonal the yttrium member of the same family of compounds (NaY(MoO₄)₂) (Figure 12c). From here, this sample will be named as "Spheres 23 nm".

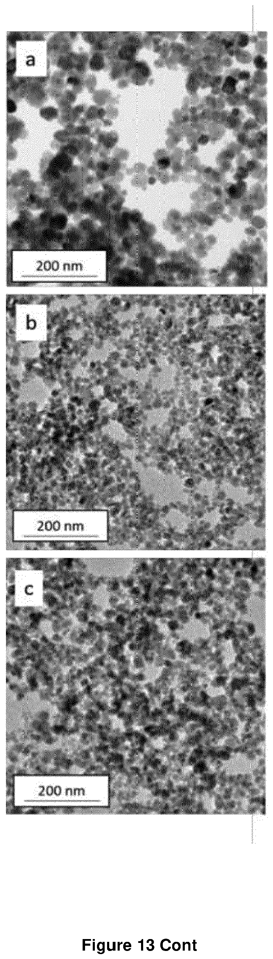

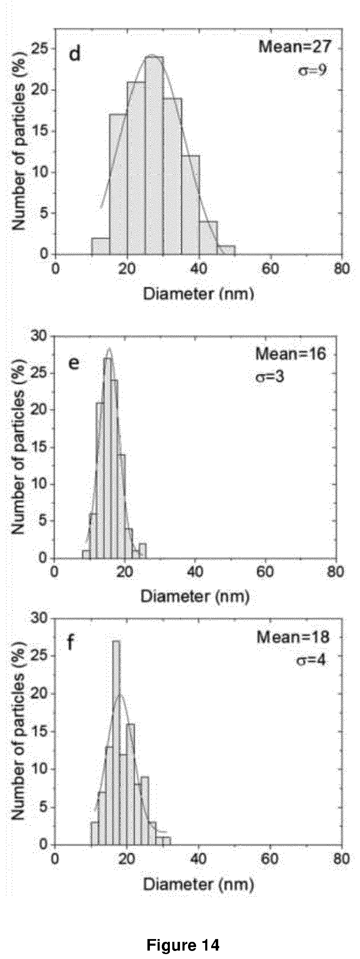

DLS measurements conducted for an aqueous dispersion of such NPs (pH = 4.6) indicated a mean hydrodynamic diameter (dₕ) of 59 nm (Figure 12d), which suggested that the particles were slightly aggregated. Nevertheless, such value of dₕ was much lower than 100 nm, which is considered as the upper limit for in vivo applications. Attempts to change particle size by altering the precipitation kinetic either changing the MoO₄²⁻/Dy³⁺mole ratio or the starting precursors total concentration or failed since although a certain variation of mean size (in the 16-27 nm range) was detected, the obtained NPs presented more irregular shape and/or broader size distribution (Figure 13).

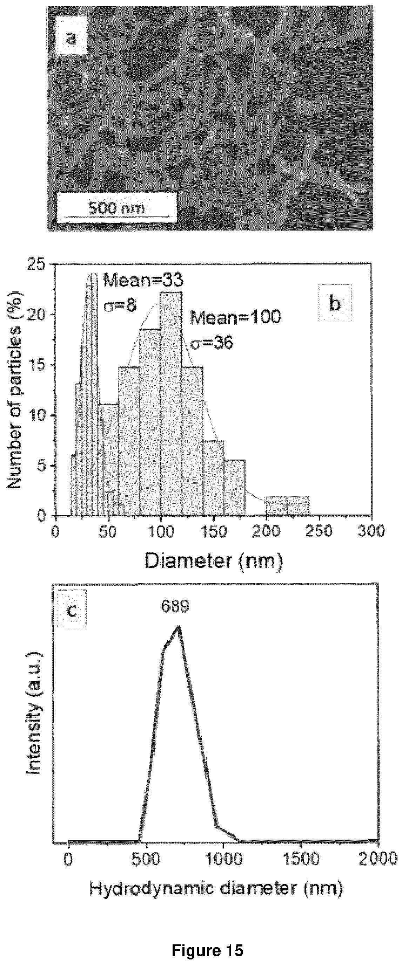

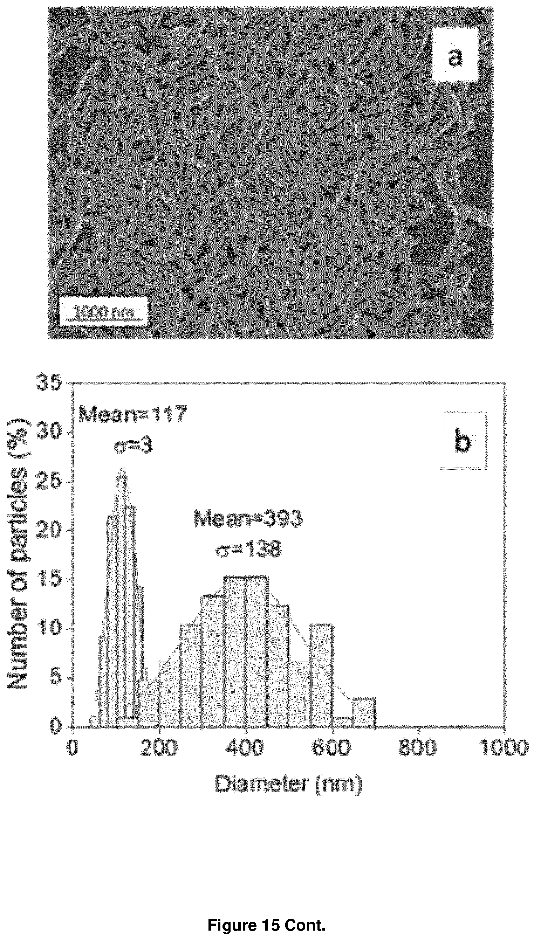

In order to obtain NPs with a shape different from spheres, a strategy consisting of the addition of a complexing agent to the starting solutions was implemented, since the formation of a metal complex before precipitation alters the reaction kinetics through changes in the nature of the soluble species precursor to precipitation, which may induce changes in particle shape. In particular, we selected tartrate anions supplied by tartaric acid (TA) for such purpose, given the proved ability of these anions to form complexes with Ln cations having different stoichiometry in aqueous solutions. As a starting point, we prepared an aqueous solution with the same Dy³⁺ and MoO₄²⁻concentrations used for the synthesis of the nanospheres, to which we added a certain amount of TA (0.04 M), and this solution was aged under the same conditions (120 ºC for 20 h in a conventional oven). In effect, by so doing, the morphology of the particles changed to rod-like (Figure 14a), although the long rod dimension was above 100 nm and the size distribution was broad (Figure 14b). Moreover, these particles were strongly aggregated in aqueous dispersions as revealed by the higher dₕ value (Figure 14c) as compared with particle size. We found, however, that by increasing aging temperature to 220 ºC and changing the heating mode (from a conventional oven to a microwave-assisted reactor), highly uniform ellipsoidal particles were obtained (Figure 15a) whose mean longest and shortest axes were 393 nm (α = 138) and 117 nm (α = 3), respectively (Figure 15b).

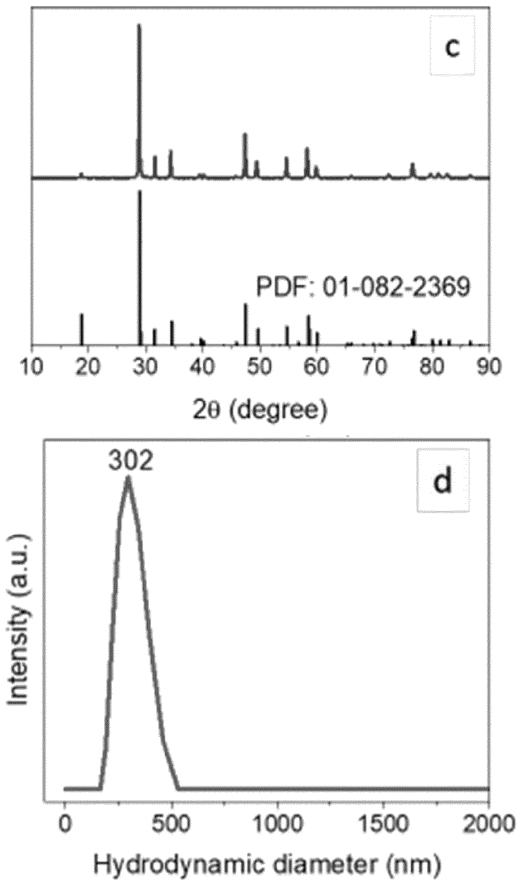

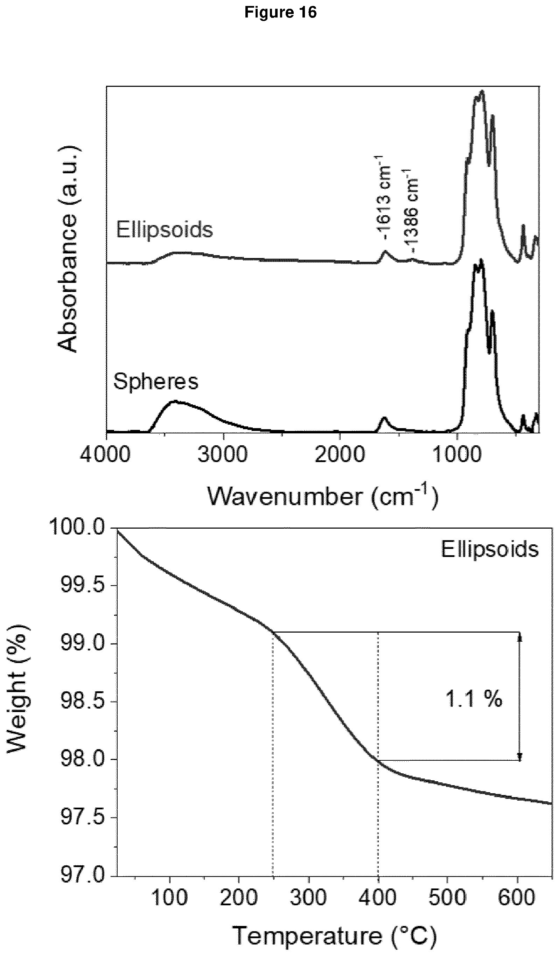

These ellipsoids consisted of tetragonal NaDy(MoO₄)₂ (Figure 15c) and were free of aggregation in aqueous dispersions (pH = 7.2) as indicated by their dₕ (302 nm), which was in between the longest and shortest particle dimensions (Figure 15d). It should be noted that after mixing the different reagents, the solutions were transparent in the presence of TA, whereas in the absence of this additive, they appeared slightly turbid, indicating an instantaneous precipitation process. This finding clearly suggests the formation of the Dy³⁺-tartrate complex, which has to be thermally decomposed on heating to induce precipitation. Such kinetics effect may account for the observed particle shape change when adding TA. Nevertheless, the complexing anions may also selectively adsorb on certain crystallographic planes blocking their growth, which may also result in anisometric particles. In order to gain information on the role played by the tartrate anions in the formation of our ellipsoidal particles, FTIR spectroscopy, and TGA measurements were conducted. The FTIR spectrum of the latter particles displayed bands in the 1630-1300 cm⁻¹ region (Figure 16 top), which can be attributed to the vibrations of carboxylate groups of tartrate anions. This assignment is also supported by the absence of these bands in the spectrum of the spherical NPs, which are synthesized in the absence of such anions (Figure 16 top). Moreover, TGA measurements revealed a 1.1 % weight loss in the 250-400 ºC range for the ellipsoidal particles (Figure 16 bottom), which may be ascribed to the decomposition of the tartrate species. Therefore, the presence of these entities most probably adsorbed on the surface of the ellipsoids may be also responsible for the role that the tartrate anions play in determining particle morphology.

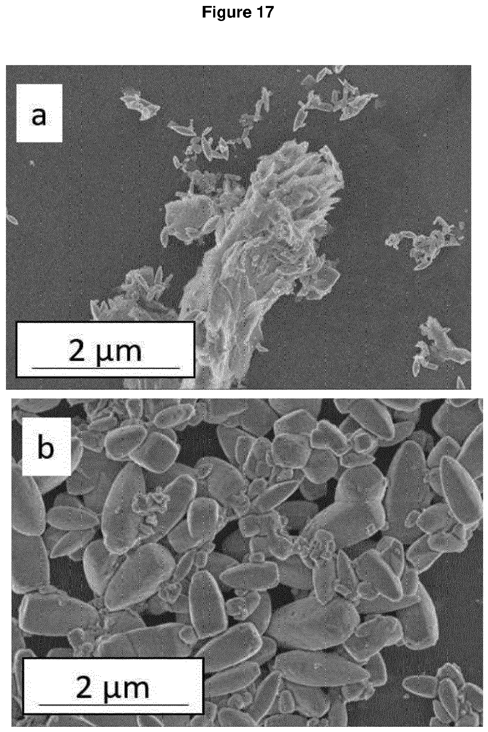

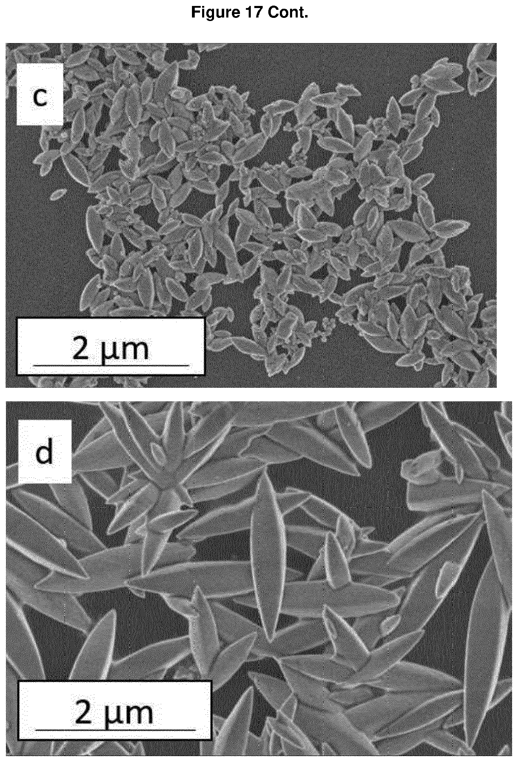

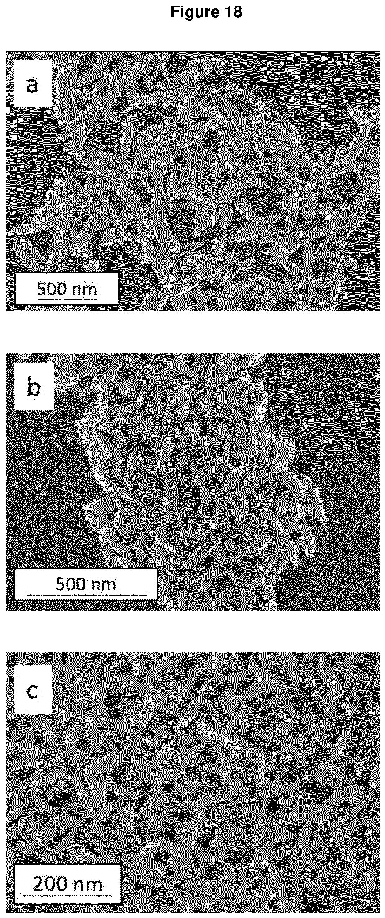

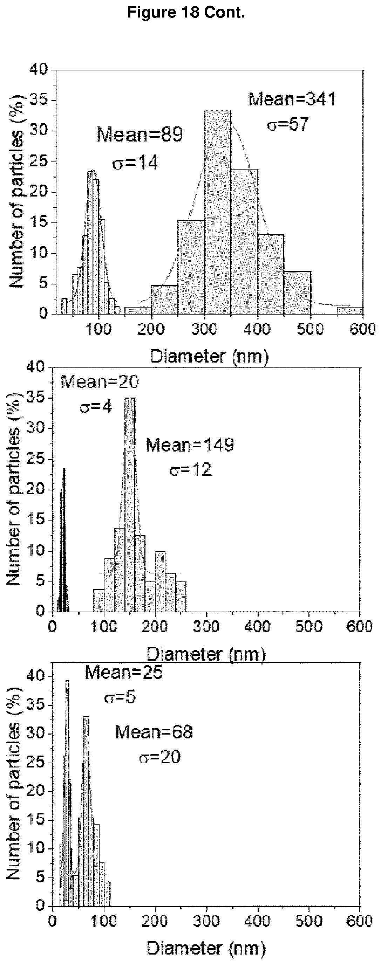

As discussed above, the NPs size required for in vivo applications must be lower than 100 nm. Therefore, several experiments were conducted to decrease the size of the ellipsoidal particles. Under the same aging conditions and TA/Dy ratio, we varied the molybdate/Dy ratio or the total concentration of the Dy and molybdate precursors keeping constant the molybdate/Dy ratio, resulting in more heterogeneous precipitates (Figure 17). The second designed strategy was to increase the viscosity of the solvent by adding different amounts of EG to water aiming to slow down the diffusion processes required for nucleation and particle growth. This approach was successful since it gave rise to a progressive decrease of the size of the ellipsoid as increasing the amount of EG, finally reaching the nanometer size range (68 x 20 nm) for a water/EG volumetric ratio of 4/6 (Figure 18). From here, the later sample will be named as "Ellipsoids 68 nm".

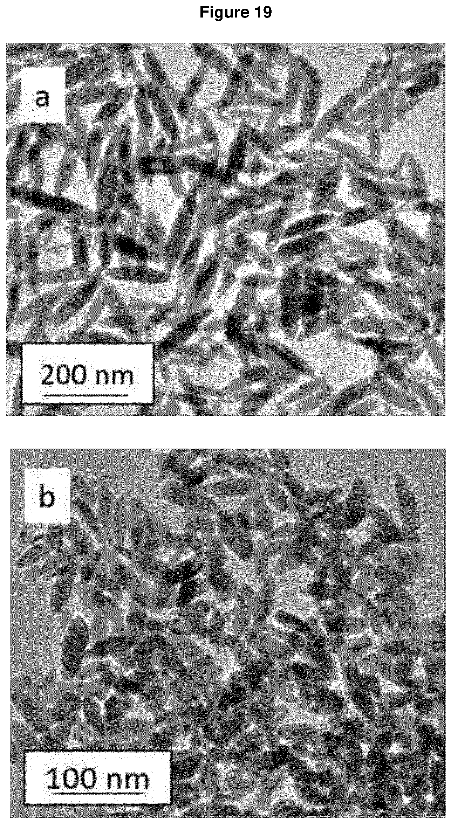

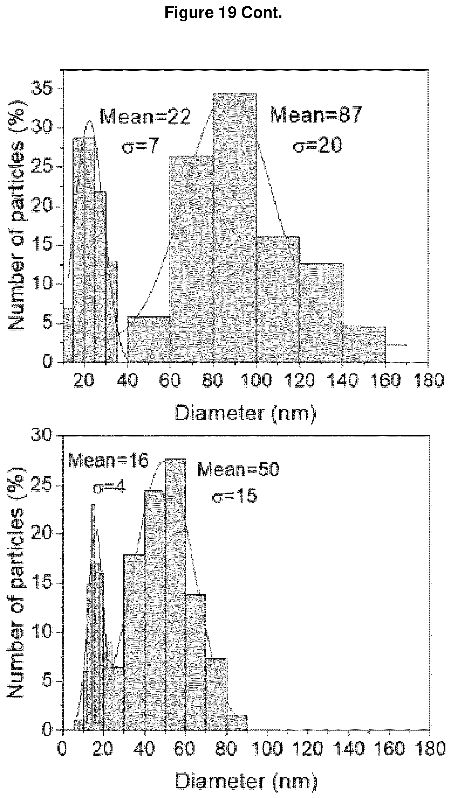

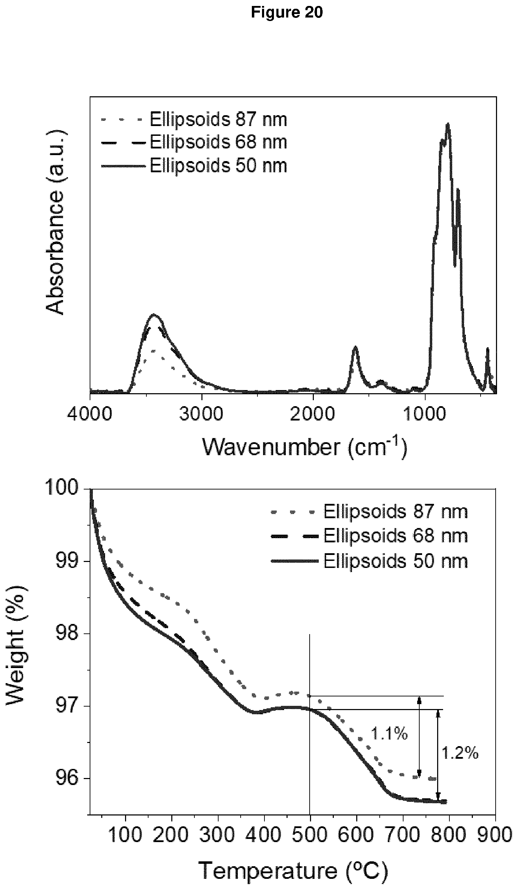

The NPs size could also be modulated by changing the amount of TA added. Thus, for the later water/EG ratio, an increase of the dimensions of the ellipsoid from 68 x 20 nm to 87 x 22 nm was observed when lowering the TA concentration (from 0.04 to 0.02 M), whereas the size decreased to 50 x 16 nm when the amount of TA was increased up to 0.06 M (Figure 19). Hereafter, these samples will be named as "Ellipsoids 87 nm" and "Ellipsoids 50 nm", respectively. It should be noticed that the amount of TA adsorbed on the NPs surface only increased slightly as increasing the raw TA amount as shown by FTIR and TGA (Figure 20), which might suggests that its role in tailoring particle size is not related to its capping ability but rather to the influence of TA concentration on the relative amount of the different Ln-tartrate complexes of a given stoichiometry formed previously to precipitation, which must also affect to nucleation and particle growth processes.

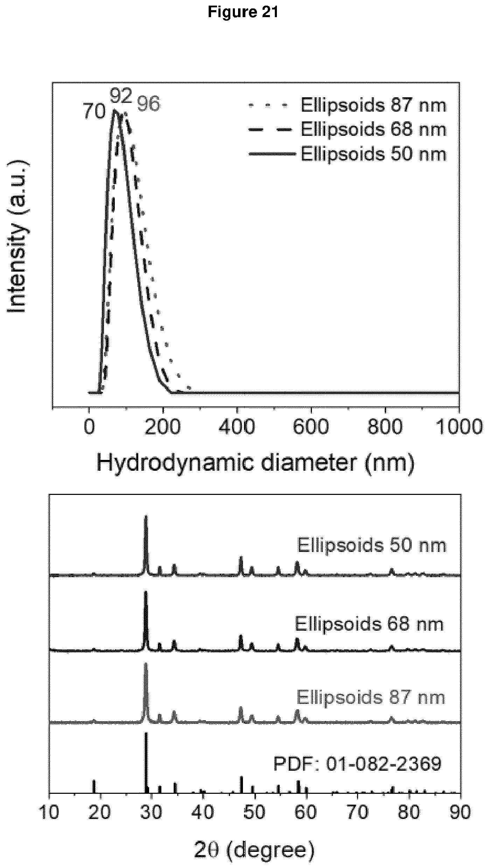

In all cases, the NPs obtained using different TA amounts were well dispersed in aqueous suspension (pH ~ 6.5) as indicated by their dₕ as determined by DLS, which was < 100 nm (Figure 21, top) and consisted of tetragonal NaDy(MoO₄)₂, as indicated by XRD (Figure 21, bottom). Since the size of these three samples was below 100 nm, they were selected to evaluate the effects of NPs size on their relaxivity.

### Relaxometric properties

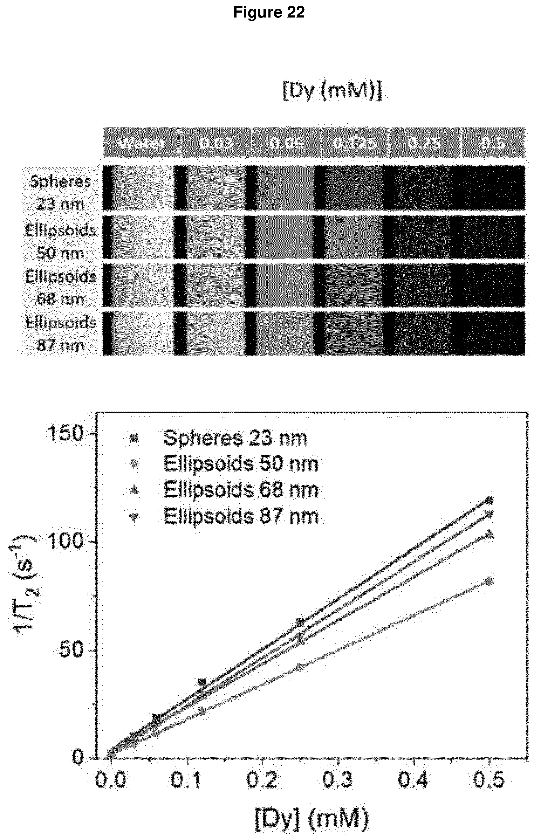

In vitro T₂- weighted phantom MRI images measured at 9.4 T for aqueous dispersions of our NaDy(MoO₄)₂ NPs having different morphology and size are shown in Figure 22 (top) as a function of the NPs content. It can be clearly observed that the images become progressively darker as increasing NPs concentration, indicating that all samples act as very efficient high field MRI T₂ CAs. For a deeper analysis of the relaxometric properties of these samples, the inverse of the T₂ values obtained from these images is plotted in Figure 22 (bottom) vs. Dy concentration.

The values of the transverse relaxivity estimated as the slope of the straight lines resulting from such plots are shown in Table 1, along with the NPs dimensions and dₕ values.

**Table 1. Values of the transverse relaxivity obtained for aqueous suspensions of the spherical and ellipsoidal NaDy(MoO₄)₂ NPs with different sizes and hydrodynamic diameter (dₕ).**

| Shape | Size/TEM (nm) | dₕ/DLS (nm) | r2 (mM⁻¹s⁻¹) |
|---|---|---|---|
| Spherical | 23 | 59 | 230 |
| Ellipsoidal | 50x16 | 92 | 160 |
| Ellipsoidal | 68x20 | 70 | 198 |
| Ellipsoidal | 87x22 | 96 | 220 |

It can be first observed that for the ellipsoidal particles, *r*₂ increased from 160 to 220 mM⁻¹S⁻¹ as increasing size from 50x16 to 87x22 nm. The *r₂* value measured for our spherical NPs is higher (230 mM⁻¹s⁻¹) than that of the ellipsoidal ones (≤ 220 mM⁻¹S⁻¹) in spite of the smaller size of the former (Table 1).

Finally, it is worth mentioning that the r2 values obtained for our NPs are higher or similar to those previously reported for other Dy-based systems, (Norek, M.; Kampert, E.; Zeitler, U.; Peters, J. A. Tuning of the size of DY2O3 nanoparticles for optimal performance as an MRI contrast agent. Journal of the American Chemical Society 2008, 130 (15), 5335-5340; Zhang, X.; Blasiak, B.; Marenco, A. J.; Trudel, S.; Tomanek, B.; van Veggel, F. C. J. M. Design and Regulation of NaHoF4 and NaDyF4 Nanoparticles for High-Field Magnetic Resonance Imaging. Chemistry of Materials 2016, 28 (9), 3060-3072; and Das, G. K.; Johnson, N. J. J.; Cramen, J.; Blasiak, B.; Latta, P.; Tomanek, B.; van Veggel, F. NaDyF4 Nanoparticles as T-2 Contrast Agents for Ultrahigh Field Magnetic Resonance Imaging. Journal of Physical Chemistry Letters 2012, 3 (4), 524-529), which supports its suitability as high field MRI CAs.

### Colloidal stability and functionalization

To assess the colloidal stability of the developed probes under physiological conditions (PBS at pH =7.4), we selected the sample having the highest relaxivity, which was that consisting of spheres (sample Spheres 23 nm) as well as that composed by ellipsoids with the size closer to that of the spheres (sample Ellipsoids 50 nm) for comparative purposes.

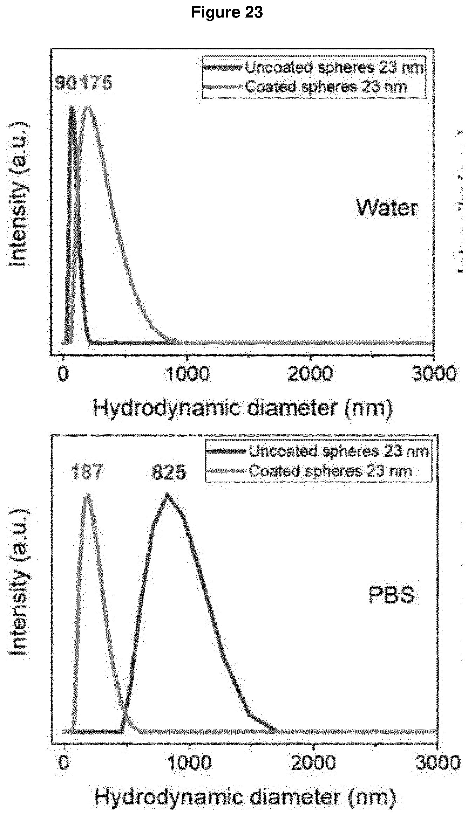

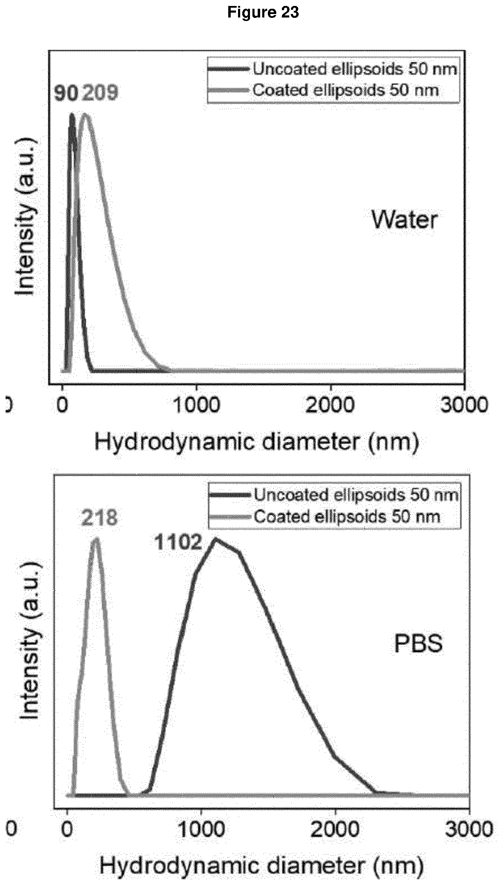

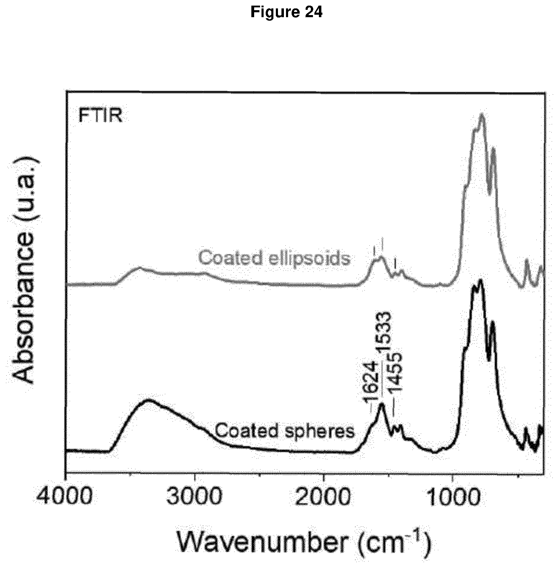

As observed in Figure 23, the dₕ value obtained from the DLS curve for these samples in PBS was much higher (> 800 nm) than in water. These results indicated that the particles were strongly aggregated under such physiological conditions. To improve colloidal stability, a strategy was envisaged consisting of coating them with two polyelectrolyte layers using the layer-by-layer approach according to the protocol described in the experimental section. Since the value of zeta potential for the spheres (-30 mV) and the ellipsoids (-28 mV) in aqueous suspensions was negative, we selected a positively charged polymer (PAH) as the first layer to favour the electrostatic interaction between both entities. In effect, the zeta potential value of the PAH coated NPs changed to +49 mV, for the spheres, and +57 mV, for the ellipsoids. This charge reversal suggests the success of the coating procedure. With the same strategy, a negatively charged polymer (PAA) was selected for the second layer and, as expected, a negative zeta potential value was then measured for both, spheres (-40 mV) and ellipsoids (-30 mV). The presence of both polymers on the nanospheres surface was confirmed by FTIR spectroscopy. Thus, the FTIR spectra obtained for the coated particles (Figure 24) displayed bands in the 1400-1650 cm⁻¹ region overlapped with that due to water at about 1620 cm⁻¹, which can be attributed to the vibrational modes of the N-H (1624 cm⁻¹ and 1455 cm⁻¹) and COO- groups (1553 cm⁻¹) of the PAH and PAA molecules, respectively, confirming the adsorption of these polymers on the NPs surface. Consequently, the NPs colloidal stability of both, the spherical and ellipsoidal NPs in the selected physiological medium was markedly improved since the dₕ values obtained for the NPs in PBS suspensions (187 nm for the spheres and 218 nm for the ellipsoids) (Figure 23) were much lower than those obtained for the uncoated NPs (> 800 nm).

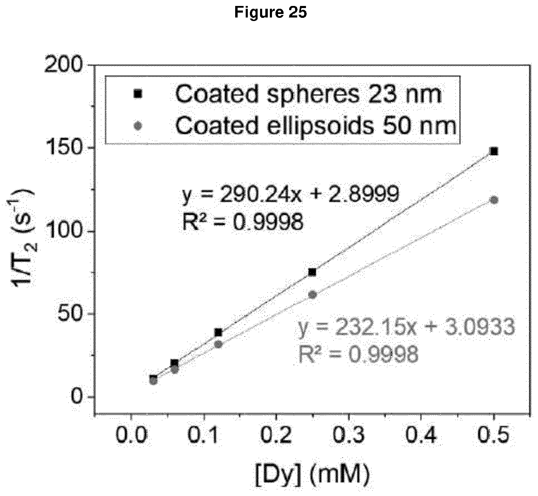

The effects of the coating layers on the relaxometric properties were also evaluated. It was found the *r₂* value increased (from 230 mM⁻¹s⁻¹ to 290 mM⁻¹s⁻¹ for the spheres and from 160 mM⁻¹s⁻¹ to 232 mM⁻¹s⁻¹ for the ellipsoids) (Figure 25) suggesting a better performance of the coated NPs as high field MRI CAs. This behaviour may be ascribed to the higher value of dh obtained for aqueous dispersions of the coated NPs used for relaxivity measurements (175 nm for the spheres and 218 nm for the ellipsoids) (Figure 23) when compared with the uncoated ones (59 nm) (Figure 12d), since, as predicted by the outer-sphere relaxation theory, *r₂* must increase with dₕ.

### Cell viability

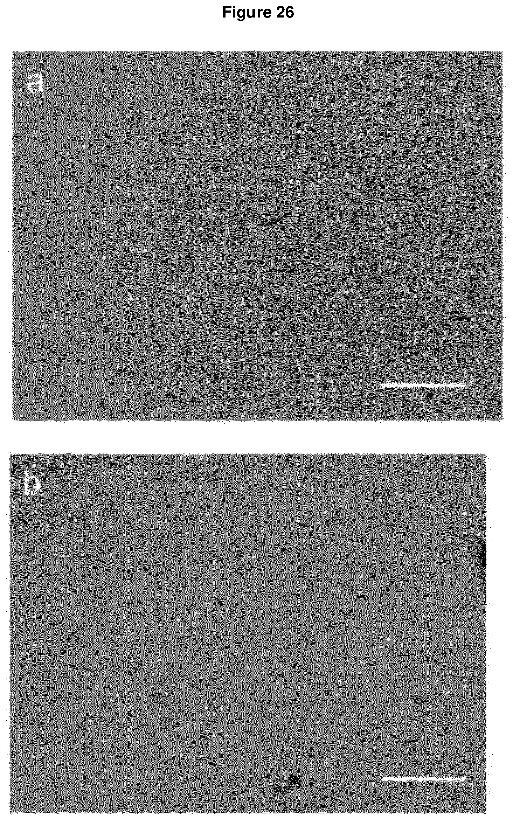

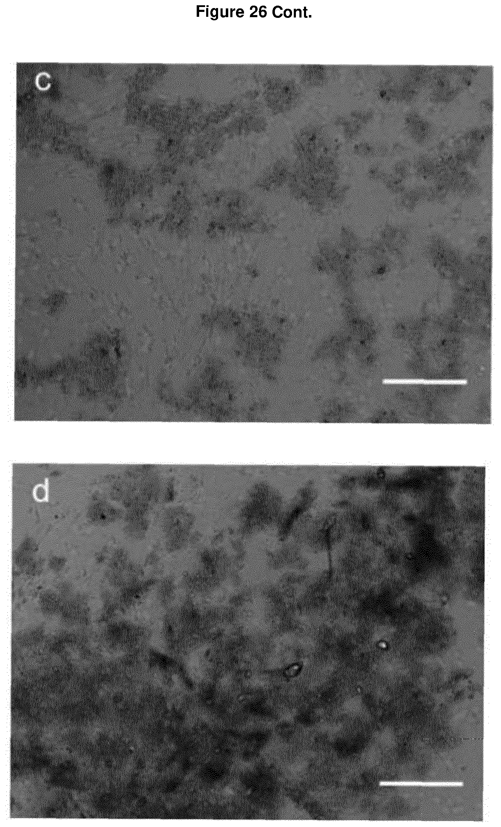

The biosafety studies were performed for the coated samples meeting the colloidal stability criteria as above discussed, using HFF-1 human foreskin fibroblasts. In order to get a deep evaluation of the cytotoxic response, several parameters were tested, such as cell morphology, induction of necrotic/late apoptotic cells mitochondrial activity, and cell uptake. First, it was found that when cells were exposed to both, spherical and ellipsoidal NPs at concentrations up to 100 mM (referred to Dy³⁺), no appreciable morphological change was appreciated (Figure 26 a-d).

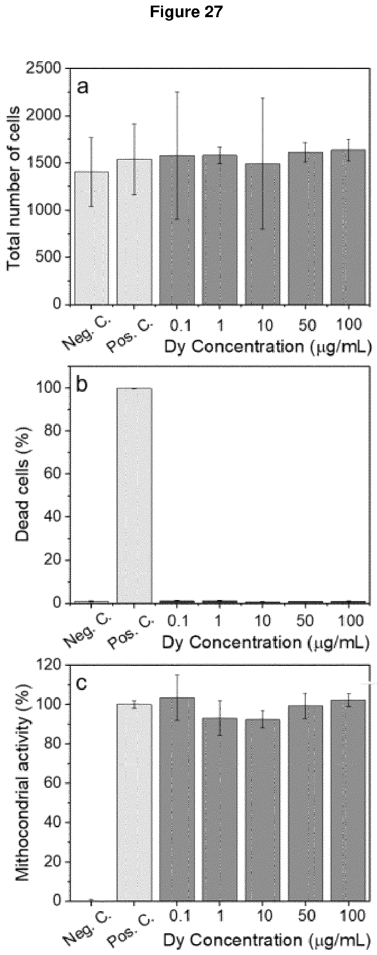

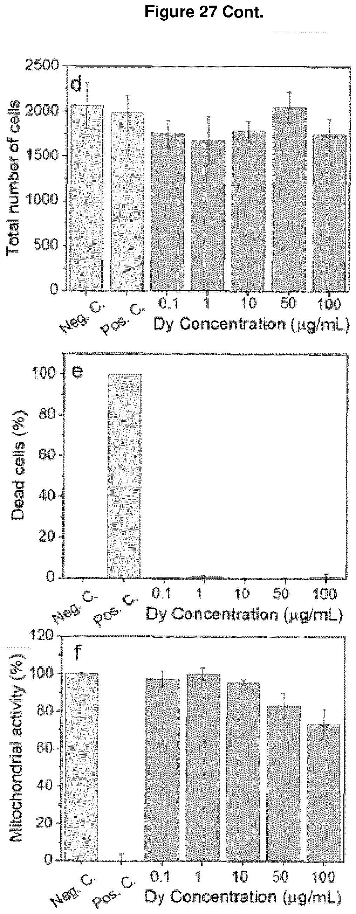

Moreover, for both kinds of NPs, cellular necrosis, evaluated by counting the total number of cells in the "live-dead" assay (Figure 27a, d), can also be discarded since no statistically significant decrease (p < 0.05) in cell number was produced for any of the concentrations tested (≤ 100 mM). Likewise, no statistically significant difference (p < 0.05) was observed between cells exposed to any concentration of NPs and negative control cells, meaning that there were no apoptotic cells (Figure 27b, e). Finally, the potential cytotoxicity of the NPs was also evaluated using the MTT assay. Cell survival was close to 100 % for every NPs concentration up to 100 mM Dy (Fig. 27c, f). These results clearly indicate negligible toxicity effects under the assayed conditions for the here synthesized and functionalized NPs, irrespective of their size and shape.

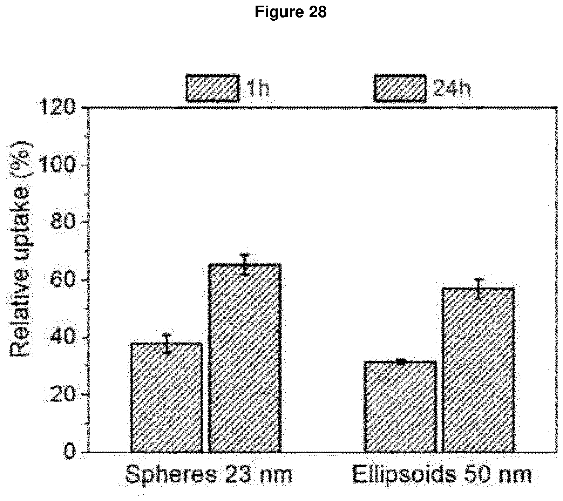

Cell uptake was also quantitatively evaluated in HFF-1 (Figure 28). One hour after the addition of 10 µg of NPs, which corresponds to 0.2 mM Dy, to culture wells containing 1 x 10⁴ cells, approximately 40% of the NPs had been internalized, increasing up to ~ 60 % after 24h. No significant differences in uptake were observed between spheres and ellipsoids, which may be related to the similar Dₕ exhibited by both NPs after coating. These results indicate that the NPs are well tolerated by cells, giving stronger support to their high biocompatibility.

### Chemical stability

Finally, we analyzed the chemical stability under conditions that may mimic the physiological medium (in PBS buffer at 37ºC) of the spherical NPs colloidally stabilized by coating with polyelectrolyte layers, taken as a case of study. It was found that this sample was chemically stable at least for 1 day, since only a 2% of Mo ions was released from the NPs after this treatment according to the ICP analyses. Therefore, the here developed NaDy(MoO₄)₂ based CAs show the required chemical stability for their use as high field MRI CAs.

## Claims

1. Nanoparticles comprising
• a core consisting of a dysprosium compound, said dysprosium compound is selected from DyPO₄ or NaDy(MoO₄)₂,
• and a coating of polyacrylic acid,
wherein the DyPO₄ nanoparticles are cubic-like and have an average value of the face diagonal ranging from 23 nm to 57 nm, and
wherein the NaDy(MoO₄)₂ nanoparticles
• are spherical and have a size ranging from 10 nm and 30 nm,
• or are ellipsoidal and have a first axis ranging from 40 nm to 95 nm and a second axis ranging from 10 nm to 30 nm.

2. Nanoparticles according to claim 1, which have an average value of the face diagonal ranging from 23 nm to 57 nm and comprise
• a core of cubic-like DyPO4,
• a first coating of polyacrylic acid on the surface of the core of cubic-like DyPO4,
• and a second coating of polyethylene glycol on the surface of the first coating.

3. Nanoparticles according to claim 1, which have a size ranging from 10 nm and 30 nm and comprise
• a core of spherical NaDy(MoO₄)₂,
• a first coating of poly(allylamine hydrochloride) positive charged polyelectrolyte on the surface of the core of spherical NaDy(MoO₄)₂,
• and a second coating of polyacrylic acid negative charged polyelectrolyte on the surface of the first coating.

4. Nanoparticles according to any of claims 1 or 3, wherein the spherical NaDy(MoO₄)₂ nanoparticles have a size ranging from 16 nm and 23 nm.

5. Nanoparticles according to claim 1, which have a first axis ranging from 40 nm to 95 nm and a second axis ranging from 10 nm to 30 nm and comprise
• a core of ellipsoidal NaDy (MoO₄)₂,
• tartaric anions adsorbed in the surface of the core of NaDy(MoO₄)₂
• a first coating of poly(allylamine hydrochloride) positive charged polyelectrolyte on the surface of the tartaric anions,
• and a second coating of polyacrylic acid negative charged polyelectrolyte on the surface of the first coating.

6. Nanoparticles according to claim 5, wherein the ellipsoidal NaDy(MoO₄)₂ nanoparticles have a first axis ranging from 50 nm to 87 nm and a second axis ranging from 16 nm to 22 nm.

7. A process for obtaining the cubic-like DyPO₄ nanoparticles according to claim 1, **characterized in that** it comprises the following steps:
a) aging, in a microwave oven, a mixture of a butanol solution of dysprosium nitrate and phosphoric acid at a temperature ranging between 120 ºC and 180 ºC for a period of time ranging between 45 min and 90 min, wherein the concentration of phosphoric acid is ranging between 0.075 M and 0.30 M in the mixture, and
b) preparing an aqueous suspension of the product obtained in step (a) and adding poly(acrylic acid) in a concentration between 2 mg/ml and 10 mg/ml adjusting the pH to a value ranging between 9 and 11.

8. A process for obtaining the cubic-like DyPO₄ nanoparticles according to claim 2, **characterized in that** it comprises the following steps:
a) aging, in a microwave oven, a mixture of a butanol solution of dysprosium nitrate and phosphoric acid at a temperature ranging between 120 ºC and 180 ºC, preferably between 140 ºC and 160 ºC, for a period of time ranging between 45 min and 90 min, wherein the concentration of phosphoric acid is ranging between 0.075 M and 0.30 M in the mixture, and
b) preparing an aqueous suspension of the product obtained in step (a) and adding poly(acrylic acid) in a concentration between 1 mg/ml and 20 mg/ml, preferably in a concentration between 2 mg/ml and 10 mg/ml, adjusting the pH to a value ranging between 9 and 11, and
c) preparing a suspension of the nanoparticles obtained in step (b) in a borate buffer, wherein said borate buffer comprises an *N*-ethyl-*N'*-(3-(dimethylamino)propyl)carbodiimide (EDC) solution and an *N-*hydroxysuccinimide (s-NHS) solution, and adding a borate buffer PEG solution in a concentration ranging between 5 mg/ml and 20 mg/ml, preferably in a concentration of 10 mg/ml.

9. A process for obtaining the spherical NaDy(MoO₄)₂ nanoparticles according to any of claims 3 or 4 **characterized in that** it comprises the following steps:
a) aging, in a conventional oven, a mixture of an aqueous and ethylene glycol solution of sodium molybdate and an ethylene glycol solution of dysprosium chloride at a temperature ranging between 110 ºC and 130 ºC for a period of time ranging between 12 h and 25 h, wherein the concentration of Dy⁺³ is between 0.01 M and 0.04 M in the mixture, preferably at a concentration of Dy⁺³ of 0.02 M in the mixture, and wherein the concentration of MoO₄²⁻ is ranging between 0.01 M and 0.04 M in the mixture, preferably at a concentration of MoO₄²⁻ of 0.02 M in the mixture,
b) mixing an aqueous solution comprising NaCl aqueous solution and poly(allylamine hydrochloride) with an aqueous dispersion of the nanoparticles obtained in step (a) and sonicating in ice water, and
c) mixing an aqueous solution comprising NaCl aqueous solution and polyacrylic acid with the nanoparticles obtained in step (b) and sonicating in ice water.

10. A process for obtaining the ellipsoidal NaDy(MoO₄)₂ nanoparticles according to any of claims 5 or 6 **characterized in that** it comprises the following steps:
a) aging, in a microwave oven, a mixture of an aqueous and ethylene glycol solution of sodium molybdate, an ethylene glycol solution of dysprosium chloride and tartaric acid at a temperature ranging between 210 ºC and 230 ºC for a period of time ranging between 20 min and 60 min, wherein the concentration of Dy⁺³ is ranging between 0.01 M and 0.04 M in the mixture, preferably at a concentration of Dy⁺³ of 0.02 M in the mixture, wherein the concentration of MoO₄²⁻ is ranging between 0.1 M and 0.4 M in the mixture, preferably at a concentration of MoO₄²⁻ of 0.2 M in the mixture and wherein the concentration of tartaric acid is between 0.01 M and 0.1 M in the mixture, preferably the concentration of tartaric acid is ranging between 0.02 M and 0.06 M in the mixture,
b) mixing an aqueous solution comprising NaCl aqueous solution and poly(allylamine hydrochloride) with an aqueous dispersion of the nanoparticles obtained in step (a) and sonicating in ice water, and
c) mixing an aqueous solution comprising NaCl aqueous solution and polyacrylic acid with the nanoparticles obtained in step (b) and sonicating in ice water.

11. Nanoparticles according to any of claims 1 to 6 for use as a contrast agent for imaging blood flow in magnetic resonance imaging.

12. Nanoparticles according to claim 11 for use as a contrast agent for imaging blood flow in ultra-high-field magnetic resonance imaging operating at static magnetic fields of 7 T or higher.

13. Use of the nanoparticles according to any of claims 1 to 6, as a contrast agent for imaging blood flow in magnetic resonance imaging.

14. Use of the nanoparticles according to claim 13 as a contrast agent for imaging blood flow in ultra-high-field magnetic resonance imaging operating at static magnetic fields of 7 T or higher.

15. A method of imaging blood flow in magnetic resonance imaging, preferably in ultra-high-field magnetic resonance imaging operating at static magnetic fields of 7 T or higher, using the nanoparticles according to any of claims 1 to 6.
